# EUROPEAN PATENT APPLICATION

(11) **EP 3 620 474 A1**
(43) Date of publication of application: **11.03.2020**
(21) Application number: 17840761.5
(22) Date of filing: 10.04.2017
(51) Int. Cl.: C07K 19/00, C12N 15/62, C12N 15/63, C12N 15/85, C12N 5/10, A61K 38/18, A61P 3/04, A61P 3/06, A61P 3/10

(54) **HUMAN FIBROBLAST GROWTH FACTOR 21 (HFGF21) FUSION PROTEIN, PREPARATION METHOD THEREFOR, AND USE THEREOF**

(30) Priority: 19.08.2016 CN 201610694914
(71) Applicant: Ampsource Biopharma Inc., Shanghai 201203 (CN); Shandong Newtime Pharmaceutical Co., Ltd, Linyi, Shandong 273400 (CN); Lunan Pharmaceutical Group Corporation, Linyi, Shandong 276005 (CN)
(72) Inventor: DONG, Zhao, Shanghai 201203 (CN); ZHOU, Chi, Shanghai 201203 (CN); FENG, Xiong, Shanghai 201203 (CN); LI, Zirui, Shanghai 201203 (CN); LI, Yuanli, Shanghai 201203 (CN); LI, Qiang, Shanghai 201203 (CN)
(74) Representative: Finnegan Europe LLP
(86) International application number: PCT/CN2017/079871
(87) International publication number: WO 2018/032785

(57) **Abstract**

A fusion protein of hFGF21 or its analogs having improved pharmaceutical properties, and use of the fusion protein in preparing medicines for treating diseases, such as diabetes, obesity, non-alcoholic fatty liver disease, dyslipidemia, and/or metabolic syndrome.

SEQUENCE LISTING

<110>  AMPSOURCE BIOPHARMA SHANGHAI INC.

<120>  HUMAN FIBROBLAST GROWTH FACTOR 21 (HFGF21) FUSION PROTEIN,
       PREPARATION METHOD THEREFOR, AND USE THEREOF

<130>  201703

<160>  9

<170>  PatentIn version 3.3

<210>  1
<211>  209
<212>  PRT
<213>  Amino acid sequence of wild-type human FGF21

<400>  1

<210>  2
<211>  33
<212>  PRT
<213>  Amino acid sequence of hCG ¦Â (113-145)

<400>  2

Pro Arg Phe Gln Asp Ser Ser Ser Ser Lys Ala Pro Pro Pro Ser
1               5                   10                  15

Leu Pro Ser Pro Ser Arg Leu Pro Gly Pro Ser Asp Thr Pro Ile Leu
                20                  25                  30

Pro Gln

<210>  3
<211>  227
<212>  PRT
<213>  Amino acid sequence of vFc¦Ã1

<400>  3

Asp Lys Thr His Thr Cys Pro Pro Cys Pro Ala Pro Glu Val Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Ser Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly Lys
225

<210> 4
<211> 223
<212> PRT
<213> Amino acid sequence of vFc¦Ã2-1

<400> 4

Val Glu Cys Pro Pro Cys Pro Ala Pro Pro Val Ala Gly Pro Ser Val
1               5                   10                  15

Phe Leu Phe Pro Pro Lys Pro Lys Asp Thr Leu Met Ile Ser Arg Thr
            20                  25                  30

Pro Glu Val Thr Cys Val Val Val Asp Val Ser His Glu Asp Pro Glu
        35                  40                  45

Val Gln Phe Asn Trp Tyr Val Asp Gly Val Glu Val His Asn Ala Lys
    50                  55                  60

Thr Lys Pro Arg Glu Glu Gln Phe Asn Ser Thr Phe Arg Val Val Ser
65                  70                  75                  80

Val Leu Thr Val Val His Gln Asp Trp Leu Asn Gly Lys Glu Tyr Lys
                85                  90                  95

Cys Lys Val Ser Asn Lys Gly Leu Pro Ala Ser Ile Glu Lys Thr Ile
            100                 105                 110

Ser Lys Thr Lys Gly Gln Pro Arg Glu Pro Gln Val Tyr Thr Leu Pro
        115                 120                 125

Pro Ser Arg Glu Glu Met Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Met Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Met His Glu Ala Leu
        195                 200                 205

His Asn His Tyr Thr Gln Lys Ser Leu Ser Leu Ser Pro Gly Lys
    210                 215                 220

<210>  5
<211>  223
<212>  PRT
<213>  Amino acid sequence of vFc¦Ã2-2

<400>  5

Val Glu Cys Pro Pro Cys Pro Ala Pro Pro Val Ala Gly Pro Ser Val
1               5                   10                  15

Phe Leu Phe Pro Pro Lys Pro Lys Asp Gln Leu Met Ile Ser Arg Thr
            20                  25                  30

Pro Glu Val Thr Cys Val Val Val Asp Val Ser His Glu Asp Pro Glu
        35                  40                  45

Val Gln Phe Asn Trp Tyr Val Asp Gly Val Glu Val His Asn Ala Lys
    50                  55                  60

Thr Lys Pro Arg Glu Glu Gln Phe Asn Ser Thr Phe Arg Val Val Ser
65                  70                  75                  80

Val Leu Thr Val Val His Gln Asp Trp Leu Asn Gly Lys Glu Tyr Lys
                85                  90                  95

Cys Lys Val Ser Asn Lys Gly Leu Pro Ala Pro Ile Glu Lys Thr Ile
            100                 105                 110

Ser Lys Thr Lys Gly Gln Pro Arg Glu Pro Gln Val Tyr Thr Leu Pro
        115                 120                 125

Pro Ser Arg Glu Glu Met Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Met Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Leu His Glu Ala Leu
        195                 200                 205

His Asn His Tyr Thr Gln Lys Ser Leu Ser Leu Ser Pro Gly Lys
    210                 215                 220

<210>  6
<211>  223
<212>  PRT
<213>  Amino acid sequence of vFc¦Ã2-3

<400>  6

Val Glu Cys Pro Pro Cys Pro Ala Pro Pro Val Ala Gly Pro Ser Val
1               5                   10                  15

Phe Leu Phe Pro Pro Lys Pro Lys Asp Gln Leu Met Ile Ser Arg Thr
            20                  25                  30

Pro Glu Val Thr Cys Val Val Val Asp Val Ser His Glu Asp Pro Glu
        35                  40                  45

Val Gln Phe Asn Trp Tyr Val Asp Gly Val Glu Val His Asn Ala Lys
    50                  55                  60

Thr Lys Pro Arg Glu Glu Gln Phe Asn Ser Thr Phe Arg Val Val Ser
65                  70                  75                  80

Val Leu Thr Val Val His Gln Asp Trp Leu Asn Gly Lys Glu Tyr Lys
                85                  90                  95

Cys Lys Val Ser Asn Lys Gly Leu Pro Ala Ser Ile Glu Lys Thr Ile
            100                 105                 110

Ser Lys Thr Lys Gly Gln Pro Arg Glu Pro Gln Val Tyr Thr Leu Pro
        115                 120                 125

Pro Ser Arg Glu Glu Met Thr Lys Asn Gln Val Ser Leu Thr Cys Leu
    130                 135                 140

Val Lys Gly Phe Tyr Pro Ser Asp Ile Ala Val Glu Trp Glu Ser Asn
145                 150                 155                 160

Gly Gln Pro Glu Asn Asn Tyr Lys Thr Thr Pro Pro Met Leu Asp Ser
                165                 170                 175

Asp Gly Ser Phe Phe Leu Tyr Ser Lys Leu Thr Val Asp Lys Ser Arg
            180                 185                 190

Trp Gln Gln Gly Asn Val Phe Ser Cys Ser Val Leu His Glu Ala Leu
        195                 200                 205

His Asn His Tyr Thr Gln Lys Ser Leu Ser Leu Ser Pro Gly Lys
    210                 215                 220


<210>  7
<211>  229
<212>  PRT
<213>  Amino acid sequence of vFc¦Ã4

<400>  7

Glu Ser Lys Tyr Gly Pro Pro Cys Pro Pro Cys Pro Ala Pro Glu Phe
1               5                   10                  15

Ala Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly Lys
225

<210>  8
<211>  459
<212>  PRT
<213>  Amino acid sequence of FP4I

<400>  8

His Pro Ile Pro Asp Ser Ser Pro Leu Leu Gln Phe Gly Gly Gln Val
1               5                   10                  15

Arg Gln Arg Tyr Leu Tyr Thr Asp Asp Ala Gln Gln Thr Glu Ala His
            20                  25                  30

Leu Glu Ile Arg Glu Asp Gly Thr Val Gly Gly Ala Ala Asp Gln Ser
        35                  40                  45

Pro Glu Ser Leu Leu Gln Leu Lys Ala Leu Lys Pro Gly Val Ile Gln
    50                  55                  60

Ile Leu Gly Val Lys Thr Ser Arg Phe Leu Cys Gln Arg Pro Asp Gly
65                  70                  75                  80

Ala Leu Tyr Gly Ser Leu His Phe Asp Pro Glu Ala Cys Ser Phe Arg
                85                  90                  95

Glu Leu Leu Leu Glu Asp Gly Tyr Asn Val Tyr Gln Ser Glu Ala His
            100                 105                 110

Gly Leu Pro Leu His Leu Pro Gly Asn Lys Ser Pro His Arg Asp Pro
        115                 120                 125

Ala Pro Arg Gly Pro Ala Arg Phe Leu Pro Leu Pro Gly Leu Pro Pro
    130                 135                 140

Ala Pro Pro Glu Pro Pro Gly Ile Leu Ala Pro Gln Pro Pro Asp Val
145                 150                 155                 160

Gly Ser Ser Asp Pro Leu Ser Met Val Gly Pro Ser Gln Gly Arg Ser
                165                 170                 175

Pro Ser Tyr Ala Ser Gly Ser Gly Gly Gly Gly Ser Gly Gly Gly Gly
            180                 185                 190

Ser Gly Gly Gly Gly Ser Gly Gly Gly Gly Ser Gly Gly Gly Gly Ser
        195                 200                 205

Ser Ser Ser Ser Lys Ala Pro Pro Pro Ser Leu Pro Ser Pro Ser Arg
    210                 215                 220

Leu Pro Gly Pro Ser Asp Thr Pro Ile Leu Pro Gln Val Glu Cys Pro
225                 230                 235                 240

Pro Cys Pro Ala Pro Pro Val Ala Gly Pro Ser Val Phe Leu Phe Pro
                245                 250                 255

Pro Lys Pro Lys Asp Gln Leu Met Ile Ser Arg Thr Pro Glu Val Thr
            260                 265                 270

Cys Val Val Val Asp Val Ser His Glu Asp Pro Glu Val Gln Phe Asn
        275                 280                 285

Trp Tyr Val Asp Gly Val Glu Val His Asn Ala Lys Thr Lys Pro Arg
    290                 295                 300

Glu Glu Gln Phe Asn Ser Thr Phe Arg Val Val Ser Val Leu Thr Val
305                 310                 315                 320

Val His Gln Asp Trp Leu Asn Gly Lys Glu Tyr Lys Cys Lys Val Ser
                325                 330                 335

Asn Lys Gly Leu Pro Ala Ser Ile Glu Lys Thr Ile Ser Lys Thr Lys
            340                 345                 350

Gly Gln Pro Arg Glu Pro Gln Val Tyr Thr Leu Pro Pro Ser Arg Glu
        355                 360                 365

Glu Met Thr Lys Asn Gln Val Ser Leu Thr Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Met Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Leu His Glu Ala Leu His Asn His Tyr
        435                 440                 445

Thr Gln Lys Ser Leu Ser Leu Ser Pro Gly Lys
    450                 455

<210>  9
<211>  1380
<212>  DNA
<213>  Nucleotide sequence of FP4I

<400>  9
caccctattc ccgatagctc ccccctcctg cagttcggag gccaggtgag gcagcggtac      60

ctgtacaccg acgacgctca gcagaccgaa gctcacctgg agatcaggga ggatggaacc     120

gtcggcggag ctgctgacca gtcccccgag agcctgctgc agctgaaggc cctgaagccc     180

ggagtcatcc agatcctggg cgtgaagacc tcccggtttc tgtgtcagcg gcccgatgga     240

gccctgtacg gctccctgca ttttgacccc gaggcctgta gcttcaggga gctgctgctg     300

gaagacggct acaacgtgta ccagagcgaa gctcacggac tgcccctgca cctgcctggc     360

aacaaatccc ctcacaggga ccccgctccc aggggacctg ccaggttcct gcctctgccc     420

ggactgcctc ctgctcctcc cgaacctcct ggcatcctcg ctcctcagcc ccctgatgtc     480

ggcagcagcg accctctgtc catggtcggc cccagccaag gcaggagccc ttcctacgct     540

tccggatccg gtggcggtgg ctccggtgga ggcggaagcg gcggtggagg atcaggcggt     600

ggaggtagcg gcggaggcgg tagctccagc tctagtaaag ctccccctcc ttccctgccc 660

tcaccctcaa gactgcctgg accttccgac actcccatcc tgccacaggt ggagtgccct 720

ccatgtccag caccccctgt cgcaggtcca tctgtgttcc tgtttccacc caagcctaaa 780

gaccagctga tgatctcccg caccccagaa gtcacctgtg tggtcgtgga tgtgagccat 840

gaagaccccg aggtccagtt caattggtac gtggatggcg tcgaggtgca caacgctaag 900

acaaaaccta gagaagagca gttcaactct acctttcgcg tcgtgagtgt gctgacagtc 960

gtgcaccagg actggctgaa tggcaaggag tataagtgca aagtgagcaa caaaggactg 1020

cctgcctcaa tcgaaaagac tatttccaag accaaaggac agccaagaga gccccaggtg 1080

tacaccctgc ctccaagccg cgaagagatg actaaaaatc aggtctctct gacctgtctg 1140

gtgaaggggt tttatcctag tgatatcgcc gtggaatggg agtcaaacgg tcagccagag 1200

aacaattaca agaccacacc ccctatgctg gacagcgatg ggtctttctt tctgtatagc 1260

aaactgacag tggacaagtc tcggtggcag cagggtaacg tcttctcttg cagtgtgctg 1320

cacgaagcac tgcacaatca ttacacccag aagtcactgt cactgagccc aggaaaatga 1380

## Description

### TECHNICAL FIELD

The present invention relates to a human fibroblast growth factor 21 (hFGF21) fusion protein, and use of the fusion protein for the manufacture of a medicament for the treatment of diabetes, obesity, dyslipidemia and/or metabolic syndrome.

### BACKGROUND

Fibroblast growth factors (FGFs) are a family of polypeptide growth factors exhibiting various physiological functions. The mammalian FGF family has been found to have 22 members, which are divided into 7 subfamilies. Fibroblast growth factor 21 (FGF21) belongs to the FGF19/21/23 subfamily. It is mainly expressed in the liver, but also in cells of tissues related to glycolipid metabolism, such as adipose tissue, islet β cells and muscle tissue. Unlike other FGF members, this subfamily acts in an endocrine manner, and regulates energy and bile acid homeostasis, glucose and lipid metabolism, and phosphate and vitamin D homeostasis (Moore DD et. al., Science, 2007, 316:1436-1438; Beenken et. al., Nature Reviews Drug Discover, 2009, 8: 235). The FGF21 mature protein consists of 181 amino acids. Unlike most members of the FGF family, FGF21 does not specifically bind to heparin to promote cell growth and differentiation. The C-terminus of FGF21 is closely related to its biological activity. The C-terminus of FGF21 directly binds to the co-factor β-Klotho, and then activates the FGFR receptor and downstream related signaling molecules to exert its biological effects (Yie J et. al., FEBS Lett, 2009, 583(1):19-24; and Micanovic R et. al., J Cell Physiol, 2009, 219(2): 227-234).

The metabolic regulation function of FGF21 on the body was first elucidated by Kharitonenkov et. al. FGF21 promoted the uptake of glucose in 3T3-L1 cells and human primary adipocytes by regulating the expression of glucose transporter 1 (GLUT1). Subsequent experiments showed that FGF21 significantly reduced body weight and blood glucose levels, as well as triglyceride levels in the liver and serum, and significantly affected insulin sensitivity in genetically-controlled or diet-induced obese mice (Kharitonenkov A et. al., Journal of clinical Investigation, 2005, 115(6):1627-1635; Coskun T et. al., Endocrinology, 2008, 149(12):6018-6027; Xu J et. al., Diabetes, 2009, 58(1):250-259). Administration of exogenous FGF21 to diet-induced obese mice and ob/ob mice can reverse hepatic steatosis and enhance hepatic insulin sensitivity (including reduction of hepatic glucose production and increase of hepatic glycogen content), thereby improving systemic glucose intolerance and insulin resistance (Xu J et. al., Diabetes, 2009, 58(1):250-259; Berglund ED et. al., Endocrinology, 2009,150(9):4084-4093; Xu J et. al., Am J Physiol Endocrinol Metab, 2009, 297(5)E1105-1104). In addition to the same metabolic regulation effects as described above, FGF21 also reduces the concentration of low density lipoprotein cholesterol and increases the concentration of high density lipoprotein cholesterol in diabetic monkeys (Kharitonenkov A et. al., Endocrinology, 2007,148(2):774-781). In summary, FGF21 has a significant beneficial metabolic regulation effect on obese rodent and non-human primates. FGF21, which regulates blood glucose levels independent of insulin, does not cause hypoglycemia when administered as a hypoglycemic agent in large doses. In addition, FGF21 is the only cytokine currently found in the FGF family that does not have mitogenic effects, thereby greatly reducing the risk of side effects in clinical use.

A large number of preclinical and clinical research have shown that FGF21 is a potential excellent target for the treatment of obesity, type 2 diabetes and hyperlipidemia. However, due to its own physical and chemical properties, native FGF21 is difficult to be developed into clinical therapeutic biological agents mainly because of the reasons as follows: 1. FGF21 protein has poor stability and is susceptible to protease hydrolysis or enzymatic degradation; 2. FGF21 has a very unstable conformation and is prone to aggregation. The low stability also increases the difficulty of FGF21 production in large-scale; 3. The native FGF21 has a very short half-life. Human FGF21 has an *in vivo* half-life of 0.5-1 hour in mice, and of 2-3 hours in cynomolgus monkeys (Kharitonenkov A et. al., Journal of clinical Investigation, 2005, 115:1627-1635). A variety long-acting technologies for protein have been reported to extend the *in vivo* half-life of recombinant FGF21, for example, by linking FGF21 to a PEG molecule to increase the molecule weight thereof to reduce the glomerular filtration rate and prolong the *in vivo* retention time (see patents WO2005/091944, WO2006/050247, WO2008/121563 and WO2012/066075); infusing FGF21 with a fatty acid long chain which is capable of binding to serum albumin (see WO2010/084169 and WO2012/010553); or preparing an agonist antibody that specifically binds to the FGFR or FGFR/β-klotho complex to activate the FGF/FGFR signaling pathway by mimicking the action mechanism of FGF21 (see WO2011/071783, WO2011/130417, WO2012/158704 and WO2012/170438); or infusing with a Fc fragment to improve the half-life of FGF21 (see WO2004/110472, WO2005/113606, WO2009/149171, WO2010/042747, WO2010/129503, WO2010/129600, WO2013/049247, WO2013/188181 and WO2016/114633).

In the field of long-acting technologies for protein , Fc fusion technology is the most widely used because it leads to fewer clinical side effects, for example, it is not easy to induce an allergic reaction or increase the toxicity of the drug due to the prolonged half-life. The key to the development of FGF21/Fc long-acting fusion protein drugs lies in the following points. First, whether the biological activity of FGF21 can be maintained. The C-terminus of FGF21 contains a binding site for β-Klotho, and thus fusion of Fc to the C-terminus of FGF21 results in a significant decrease in its activity, while fusion to the N-terminus retains its binding affinity to β-Klotho to the utmost extent. Therefore, in the prior art, the Fc fragment is mostly fused to the N-terminus of FGF21 (Fc-FGF21). Second, whether the fusion of Fc can significantly improve the pharmacokinetic properties of FGF21 and effectively prolong its half-life to meet the clinical requirement for administrating twice a week or even once a week. The FGF21 is highly susceptible to degradation because it contains a protease hydrolysis site at the C-terminus, and the *in vitro* activity of its hydrolyzed metabolites is reduced by nearly 200-fold (Micanovic R et. al., J Cell Physiol,2009,219(2):227-234; Yie J et. al., FEBS Lett, 2009,583(1):19-24). According to pharmacokinetic studies on Fc-FGF21, its half-life was not significantly improved because the C-terminus (especially between Pro171 and Ser172) of FGF21 in the fusion protein was exposed and was not protected by Fc, and thus it is susceptible to degradation by protease attack (Hecht, R et. al., PLoS One, 2012, 7:e49345). Third, how to reduce the risk of an immune response induced by the linker sequence or the introduced mutation site. Fourth, whether the fusion of Fc and/or the introduction of a mutation site can improve the stability of FGF21 and its polymerizability in a highly concentrated state. Therefore, for an ideal FGF21 Fc fusion protein, on the one hand, the anti-proteolytic ability of the C-terminus of FGF21 should be enhanced as well as the half-life should be significantly extended; on the other hand, the binding affinity of the C-terminus of FGF21 to β-Klotho should not be significantly reduced due to the steric effect of Fc, otherwise will result in a significant decrease in its activity; at the same time, the introduction of the fusion ligand and the linker peptide should not increase its immunogenicity, but improving its stability.

The carboxyl terminal peptide (hereinafter referred to as CTP) of the human chorionic gonadotropin (hCG) beta chain has the effect of prolonging the *in vivo* half-life of certain proteins. Thus CTP is used in fusion proteins as disclosed in some patent documents as a fusion ligand for prolonging the half-life of protein of interest. In addition, CTP can also be used as a linker mainly for linking different subunits of the same protein. For example, CTP is used as a linker between the beta and alpha subunits of follicle stimulating hormone, as disclosed in Chinese Patent Nos. CN103539860A, CN103539861A, CN103539868A, and CN103539869A. As another example, CTP is used as a linker between the beta and alpha subunits of glycoprotein hormone as disclosed in the patent WO2005058953A2.

The present inventors do not use CTP as a linker or as a half-life prolonging moiety as suggested by the prior art, but instead connect it to a flexible peptide linker (e.g., (GGGGS)n) to constitute a new linker sequence. The new linker is located between FGF21 and the half-life prolonging moiety (*e.g.,* the immunoglobulin Fc fragment, which does not contain the CTP as suggested by the prior art) to constitute a new FGF21 fusion protein, further prolonging the half-life and maintaining the biological activity and function of FGF21.

### SUMMARY

The object of the present invention is to solve the problems of short half-life and poor stability of FGF21, and to provide a highly glycosylated human FGF21 fusion protein with improved pharmaceutical properties, for example, enhanced proteolytic resistance, prolonged *in vivo* half-life and reduced aggregation.

In one aspect, the present invention provides a highly glycosylated human fibroblast growth factor 21 (hFGF21) fusion protein, which is hereinafter referred to as hFGF21 fusion protein. The fusion protein comprises, in order from the N-terminus to C-terminus, wild-type human fibroblast growth factor 21 or an analog thereof (denoted as hFGF21), a flexible peptide linker (denoted as L), at least one rigid unit comprising the carboxyl terminal peptide of human chorionic gonadotropin β subunit (hereinafter referred to as (CTP)n, wherein n is 1, 2, 3, 4, or 5) and a fusion ligand (such as an immunoglobulin and a Fc fragment thereof, an albumin or a transferrin).

In some embodiments, the fusion protein disclosed herein comprises a wild-type hFGF21 polypeptide, wherein the wild-type hFGF21 polypeptide comprises a sequence as shown in SEQ ID NO: 1 from which amino acids 1-28 (a leader peptide) are removed; or an isoform of the sequence as shown in SEQ ID NO: 1 from which amino acids 1-28 (a leader peptide) are removed and having G141S or L174P substitution.

In other embodiments, the fusion protein disclosed herein comprises an hFGF21 analog, for example, an hFGF21 analog having one or more amino acid deletions, insertions, additions or substitutions relative to its wild-type sequence, and a truncated form having one or more amino acid deletions at the N- or C-terminus. Preferably, the amino acid sequence of the hFGF21 analog is at least 70% homologous to that of wild-type hFGF21; more preferably, the amino acid sequence of the hFGF21 analog is at least 80% homologous to that of wild-type hFGF21; more preferably, the amino acid sequence of the hFGF21 analog is at least 90% homologous to that of wild-type hFGF21; and most preferably, the amino acid sequence of the hFGF21 analog is at least 95% homologous to that of wild-type hFGF21.

The flexible peptide linker is preferably non-immunogenic and can generate sufficient distance between hFGF21 and the fusion ligand to minimize the steric effects between each other. Preferably, a flexible peptide linker consisting of two or more amino acids selected from the group consisting of Gly (G), Ser (S), Ala (A) and Thr (T) is used.

Preferably, the flexible peptide linker comprises residues G and S. The length of the linker peptide plays a very important role in the activity of the fusion protein. For the purposes of the present invention, the flexible peptide linker preferably has the structural formula of (GS)a(GGS)b(GGGS)c(GGGGS)d, wherein each a, b, c, and d is an integer equal to or greater than 0, and a+b+c+d≥1.

In some embodiments of the present invention, the peptide linker is selected from the group consisting of:
(i) L1: GGGGS;
(ii) L2: GSGGGSGGGGSGGGGS;
(iii) L3: GSGGGGSGGGGSGGGGSGGGGSGGGGS;
(iv) L4: GGGGSGGGGSGGGGSGGGGSGGGGSGGGGSGGGGS;
(v) L5: GGGSGGGSGGGSGGGSGGGS;
(vi) L6: GGSGGSGGSGGS.

The CTP rigid unit is selected from a full-length sequence consisting of carboxyl-terminal amino acids 113 to 145 of human chorionic gonadotropin β subunit, or a truncated sequence thereof. Specifically, the CTP rigid unit comprises the amino acid sequence as shown in SEQ ID NO: 2 or a truncated sequence thereof.

Preferably, the CTP rigid unit contains at least 2 glycosylation sites. For example, in a preferred embodiment of the present invention, the CTP rigid unit contains 2 glycosylation sites. Illustratively, the CTP rigid unit contains N-terminal 10 amino acids of SEQ ID NO: 2, *i.e.* SSSS*KAPPPS*; alternatively, the CTP rigid unit contains C-terminal 14 amino acids of SEQ ID NO: 2, *i.e.* S*RLPGPS*DTPILPQ. As another example, in another embodiment, the CTP rigid unit contains 3 glycosylation sites. Illustratively, the CTP rigid unit contains N-terminal 16 amino acids of SEQ ID NO: 2, *i.e.* SSSS^{∗}KAPPPS^{∗}LPSPS^{∗}R. As another example, in other embodiments, the CTP rigid unit contains 4 glycosylation sites. Illustratively, the CTP rigid unit contains 28, 29, 30, 31, 32, or 33 amino acids, starting from position 113, 114, 115, 116, 117, or 118 and ending at position 145 of the human chorionic gonadotropin beta subunit. Specifically, the CTP rigid unit contains N-terminal 28 amino acids of SEQ ID NO: 2, *i.e.* SSSS^{∗}KAPPPS^{∗}LPSPS^{∗}RLPGPS^{∗}DTPILPQ. Herein, ^{∗} represents a glycosylation site. Each possibility represents a separate embodiment of the present invention.

In other embodiments, the amino acid sequence of the CTP rigid unit provided by the present invention is at least 70% homologous to that of native CTP. In other embodiments, the amino acid sequence of the CTP rigid unit provided by the present invention is at least 80% homologous to that of native CTP. In other embodiments, the amino acid sequence of the CTP rigid unit provided by the present invention is at least 90% homologous to that of native CTP. In other embodiments, the amino acid sequence of the CTP rigid unit provided by the present invention is at least 95% homologous to that of native CTP.

Illustratively, the CTP rigid unit of the present invention may preferably comprise the following sequences:
(i) CTP1: SSSSKAPPPSLPSPSRLPGPSDTPILPQ;
(ii) CTP2: PRFQDSSSSKAPPPSLPSPSRLPGPSDTPILPQ;
(iii) CTP3: SSSSKAPPPS;
(iv) CTP4: SRLPGPSDTPILPQ;
(v) CTP5: SSSSKAPPPSLPSPSR.

In some embodiments of the present invention, the fusion protein comprises one CTP rigid unit as described above.

The fusion protein of the present invention may comprise more than one, preferably 2, 3, 4 or 5, CTP rigid units as described above. In an embodiment of the present invention, the fusion protein comprises three CTP3 rigid units: SSSSKAPPPSSSSSKAPPPSSSSSKAPPPS (CTP3-CTP3-CTP3, or expressed as (CTP3)₃). In another embodiment of the present invention, the fusion protein comprises two CTP5 rigid units: SSSSKAPPPSLPSPSRSSSSKAPPPSLPSPSR (CTP5-CTP5, or expressed as (CTP5)₂).

The fusion ligand is preferably an immunoglobulin Fc fragment. More preferably, the Fc fragment is preferably selected from the group consisting of Fc fragments of human immunoglobulin IgG, IgM, IgA and variants thereof, more preferably from the group consisting of Fc fragments of human IgG1, IgG2, IgG3 and IgG4 and variants thereof. The human IgG Fc variant (denoted as vFc) comprises at least one amino acid modification in the wild-type human IgG Fc. In addition, the Fc variant is non-lytic and exhibits minimal Fc-mediated adverse side effects (ADCC and CDC effects) and/or enhanced binding affinity to the FcRn receptor.

Further, the human IgG Fc variant may be selected from the group consisting of:
(i) vFcγ1: hinge, CH2 and CH3 regions of human IgG1 with mutations Leu234Val, Leu235Ala, and Pro331Ser (the amino acid sequence as shown in SEQ ID NO: 3);
(ii) vFcγ2-1: hinge, CH2 and CH3 regions of human IgG2 with mutation Pro331Ser (the amino acid sequence as shown in SEQ ID NO: 4);
(iii) vFcy2-2: hinge, CH2 and CH3 regions of human IgG2 with mutations Thr250Gln and Met428Leu (the amino acid sequence as shown in SEQ ID NO: 5);
(iv) vFcy2-3: hinge, CH2 and CH3 regions of human IgG2 with mutations Pro331Ser, Thr250Gln and Met428Leu (the amino acid sequence as shown in SEQ ID NO: 6).
(iv) vFcy4: hinge, CH2 and CH3 regions of human IgG4 with mutations Ser228Pro and Leu235Ala (the amino acid sequence as shown in SEQ ID NO: 7).

The IgG Fc variants provided by the present invention include, but are not limited to, the five variants as described in (i) to (v), and may also be those obtained by combining or adding the mutation sites of two functional variants of the same IgG subtype. For example, the variant as described in (iv) is a new IgG2 Fc combination variant obtained by adding the mutation sites in (ii) and (iii).

The Fc variant (vFc) in the fusion protein of the present invention comprises hinge, CH2 and CH3 regions of human IgG such as human IgG1, IgG2 and IgG4. The CH2 region contains amino acid mutations at positions 228, 234, 235 and 331 (as defined by the EU numbering system). It is believed that these amino acid mutations reduce the effector functions of Fc. Human IgG2 Fc does not bind to FcyR but shows extremely weak complement activity. An Fcy2 variant with mutation Pro331Ser should have less complement activity than native Fcy2 while remain as a non-binder to FcyR. IgG4 Fc is deficient in activating the complement cascade, and its binding affinity to FcyR is about an order of magnitude lower than that of IgG1. An Fcy4 variant with mutations Ser228Pro and Leu235Ala should exhibit minimal effector functions as compared to the native Fcγ4. An Fcγ1 variant with mutations Leu234Val, Leu235Ala and Pro331Ser also should exhibit decreased effector functions than the native Fcγ1. These Fc variants are more suitable for the preparation of fusion proteins of hFGF21 and an analog thereof than native human IgG Fcs. The amino acid mutations at positions 250 and 428, as defined by the EU numbering system, increase the binding affinity of the Fc region to the neonatal receptor FcRn, thereby further prolonging the half-life (Paul R et al., J Biol Chem, 2004, 279:6213-6216). The above two types of functional variants are combined or added on each other to generate new combination variants to reduce the effector functions while prolonging the half-life. The Fc variants of the present invention contain mutations at, but not limited to, the above-described sites, and substitutions may be introduced at other sites such that Fcs have reduced effector functions and/or enhanced binding affinity to FcRn. Meantime, these mutations should not lead to reduction of the function/activity or undesirable conformational changes of the Fc variants. Common mutation sites may be found in Shields RL et al., J Biol Chem, 2001,276(9):6591-604.

In a preferred embodiment of the present invention, the fusion protein has the amino acid sequence as shown in SEQ ID NO: 8.

The fusion protein of the present invention is glycosylated. Preferably, the fusion protein is glycosylated by expressing the same in mammalian cells. More preferably, the fusion protein is glycosylated by expressing the same in Chinese hamster ovary cells.

According to another aspect of the present invention, a DNA encoding the fusion protein described above is provided. In a preferred embodiment of the present invention, the fusion protein has the DNA sequence as shown in SEQ ID NO: 9.

According to another aspect of the present invention, a vector is provided. This vector contains the DNA described above.

According to another aspect of the present invention, a host cell is provided. The host cell comprises or is transfected with the vector described above.

In a specific embodiment of the present invention, the host cell is CHO-derived cell line DXB-11.

According to still another aspect of the present invention, a pharmaceutical composition is provided. The pharmaceutical composition comprises a pharmaceutically acceptable carrier, excipient or diluent, and an effective amount of the fusion protein described above.

According to still another aspect of the present invention, there is provided use of the fusion protein for the preparation of a medicament for treating a condition associated with FGF21, such as obesity, type 1 or type 2 diabetes, pancreatitis, dyslipidemia, non-alcoholic fatty liver disease (NAFLD), non-alcoholic steatohepatitis (NASH), insulin resistance, hyperinsulinemia, glucose intolerance, hyperglycemia, metabolic syndrome, acute myocardial infarction, hypertension, cardiovascular disease, atherosclerosis, peripheral arterial disease, stroke, heart failure, coronary heart disease, kidney disease, diabetic complications, neuropathy, and disorders associated with severe inactivation or mutation in the insulin receptor.

According to another aspect of the present invention, a method for preparing or producing the fusion protein from a mammalian cell line, such as a CHO-derived cell line, is provided, which comprises the steps of:
(a) introducing a DNA encoding the fusion protein described above into a mammalian cell;
(b) screening the high-yielding cell line in step (a) which expresses more than 50 µg/10⁶ cells per 24 hours in its growth medium;
(c) growing the cell line obtained in step (b);
(d) harvesting the fermentation broth obtained in step (c) and purifying the fusion protein.

Preferably, the mammalian cell used in the step (a) is a CHO cell, more preferably, CHO-derived cell line DXB-11.

Compared with the current products, the hFGF21 fusion protein of the present invention has the following outstanding advantages:
1. Prolonged *in vivo* functional half-life. Glucose utilization levels in obese mice were significantly improved both in medium- and high-dose groups at 16h after administration of hFGF21 fusion protein to obese mice induced with high-fat diet, and the glucose utilization levels in obese mice were still significantly improved in the high-dose group at 48h after administration, indicating that the hFGF21 fusion protein has long-lasting effects on improving glucose utilization, at the same time, also demonstrating that the hFGF21 fusion protein has a significantly enhanced anti-proteolytic ability, allowing it to be less susceptible to degradation that results in activity reduction or loss.
2. Significantly prolonged *in vivo* circulating half-life. Since the protective effects of Fc, which is fused at the C-terminus of FGF21, reduces the susceptibility of FGF21 to proteolytic enzyme, the fusion protein constructed by the present invention has a significantly longer half-life than the fusion proteins in which Fc are fused at the N-terminus (Fc-FGF21) as reported in the prior art. On the other hand, the CTP rigid unit contains a glycosyl group. The negatively charged, highly sialylated CTP rigid unit is capable of resisting the clearance of the kidney, which further prolongs the half-life of the fusion protein. In a preferred embodiment of the present invention, it is demonstrated that the CTP-containing hFGF21 fusion protein has a longer *in vivo* circulating half-life and higher bioavailability than the CTP-free hFGF21 fusion protein. The pharmacokinetic data obtained after a single-dose in a preferred embodiment showed that the *in vivo* circulating half-life T_{1/2} of 3 mg/kg hFGF21 fusion protein FP4I in SD rats was 29.81 ± 1.56 h, whereas the T_{1/2} of CTP-free hFGF21 fusion protein FP4J was only 22.43 ± 1.45 h, which is greatly prolonged compared with the half-life of native hFGF21 (the *in vivo* circulating half-life in rats is 1∼1.5 h), allowing the frequency of administration to be greatly reduced.
3. In the prior art, the C-terminal fusion of Fc to FGF21 (FGF21-Fc) often leads to a significant decrease in its activity. However, the C-terminal Fc fusion protein constructed by the present invention utilizes a novel linker peptide which is consisted of a flexible peptide and a CTP rigid peptide. The CTP rigid unit can form a relatively stable stereoscopic conformation by containing a plurality of O-glycosyl side chains, which effectively isolates hFGF21 from Fc, thereby minimizing the steric hindrance effect generated by Fc and allowing FGF21 to maintain a better biological activity. For example, the hFGF21 fusion protein can effectively control the body weight of obese mice induced by high-fat diet, improve insulin resistance and hepatic steatosis, and exhibit significant inhibition of obesity. In addition, the protective effect of the CTP rigid unit glycosyl side chain can reduce the sensitivity of the linker peptide to proteases, making the fusion protein less susceptible to degradation in the junction region. Moreover, CTP is derived from native human hCG and thus is non-immunogenic and more suitable for being used as a linker peptide than a non-naturally encoded amino acid sequence.
4. Mutations such as P331S have been introduced in the fusion ligand Fc, retaining only the properties of Fc of long circulating half-life, and reducing or eliminating the ADCC and CDC effects, thereby increasing drug safety. In addition, Fc variants with enhanced binding affinity to neonatal receptor (FcRn) (such as T250Q/M428L) can further prolong the half-life of the fusion protein.
5. The hFGF21 fusion protein constructed by the present invention exhibits stronger thermal stability during preparation and storage, and has a higher expression level. At the same time, the hFGF21 fusion protein is also less likely to aggregate in highly concentrated preparations.

### Definitions

The term "human FGF21" and "hFGF21" as used in the present invention refer to a wild-type human FGF21 polypeptide as well as an analog thereof.

### Wild-type FGF21 polypeptide

The sequence of the wild-type FGF21 protein is available from the UNIPROT database with an Accession No. Q9NSA1. The precursor protein consists of 209 amino acids, including a signal peptide (amino acids 1-28) and a mature protein (amino acids 29-209).

An isoform or allelic form of wild-type FGF21 having a Pro instead of Leu in the mature protein at position 174 of SEQ ID NO: 1 of the present invention is known from US 2001012628 A1. Another isoform of wild-type FGF21 having a Ser instead of Gly at position 141 of SEQ ID NO: 1 of the present invention is known.

Another isoform having a shorter signal peptide in which Leu at position 23 of SEQ ID NO: 2 of the present invention is missing is known from WO 2003/011213 (see SEQ ID NO: 2 of WO2003/011213, having a signal peptide of 27 amino acid residues).

In the present invention, the wild-type hFGF21 comprises the sequence of the mature protein moiety (amino acids 29-209) obtained by removing the leader peptide from the sequence as shown in SEQ ID NO: 1 or an isoform thereof having substitution L174P or G141S. In addition, the full-length sequence of the precursor protein containing the signal peptide of 27 or 28 amino acids is also included.

### hFGF21 analogs

The hFGF21 analog as used in the present invention refers to a polypeptide which is or can be deduced or derived from wild-type human FGF21, in particular from SEQ ID NO: 1, by modification of the amino acid sequence thereof. Such modification, amendment or change may include substitution, deletion, and/or addition of one or more amino acids. The term "mutein" or "variant" is sometimes used herein instead of the term "analog". For example, amino acids may be added and/or deleted at the C-terminus, at the N-terminus, or internally in the amino acid sequence (an amino acid added internally in a sequence may be referred to as an insertion). Preferably amino acids are added and/or deleted at the C and/or N-terminus, more preferably at the N-terminus.

An amino acid sequence having a C-terminal or N-terminal deletion may also be referred to as a truncated sequence. The truncated hFGF21 polypeptide, including forms of N-terminal truncation or C-terminal truncation, is capable of providing an activity similar to, in some cases better than, the un-truncated form of the mature hFGF21 polypeptide, as is known in the art.

### a. N-terminal truncations

In some embodiments of the present invention, the hFGF21 analog having a N-terminal truncation is selected from a polypeptide in which 1, 2, 3, 4, 5, 6, 7, or 8 amino acid residues at the N-terminus of the mature hFGF21 polypeptide are deleted. FGF21 polypeptides having N-terminal truncations of fewer than 9 amino acid residues retain the ability of the mature FGF21 polypeptide to reduce blood glucose in an individual. Accordingly, in particular embodiments, the present invention encompasses truncated forms of the mature FGF21 polypeptide or FGF21 protein variants having N-terminal truncations of 1, 2, 3, 4, 5, 6, 7, or 8 amino acid residues.

### b. C-terminal truncations

In some embodiments of the present invention, the hFGF21 analog having a C-terminal truncation is selected from a polypeptide in which 1, 2, 3, 4, 5, 6, 7, 8, 9, 10, 11 or 12 amino acid residues at the C-terminus of the mature hFGF21 polypeptide are deleted. FGF21 polypeptides having C-terminal truncations of fewer than 13 amino acid residues exhibited at least 50% of the efficacy of wild-type FGF21 in an *in vitro* ELK-luciferase assay (Yie J. et al. FEBS Letts 583:19-24 (2009)), indicating that these FGF21 mutants retain the ability of the mature FGF21 polypeptide to reduce blood glucose in an individual. Accordingly, in particular embodiments, the present invention encompasses truncated forms of the mature FGF21 polypeptide or FGF21 protein variants having C-terminal truncations of 1, 2, 3, 4, 5, 6, 7, 8, 9, 10, 11 or 12 amino acid residues.

One example of an FGF21 analogue is the truncated form of wild-type mature FGF21 in which four N-terminal amino acid residues (HPIP) of the mature protein are removed, which is disclosed in, *e.g.,* WO 2006/065582. This truncated form is believed to stimulate glucose uptake in mouse 3T3-L1 adipocytes at the same level as the wild-type FGF21. In the present invention, the truncated form of hFGF21 has the amino acid sequence of amino acid 33-209 of SEQ ID NO: 1, designated as hFGF21 (HPIP⁻).

A further example of an FGF21 analogue is the polypeptide of SEQ ID NO:1 which has an N-terminal Met (also designated as "Met-hFGF21"). The N-terminal Met is added when hFGF21 is expressed in E. coli, see *e.g.* WO 2006/050247, Table 6.

Another example of an hFGF21 analog is a wild-type hFGF21 precursor protein as shown in SEQ ID NO: 1 in which one or more amino acids are substituted/replaced. The substitution/replacement includes, but is not limited to, Q55C, A109T, L126R, G148C, K150R, P158S, S195A, P199G and G202A.

Examples of amino acid sequences of hFGF21 analogs containing other modifications are disclosed in, for example, WO2003/061712, WO2005/091944, WO2006/028595, WO2006/028714, WO2006/065582 and WO2008/121563.

### hCG-β carboxyl terminal peptide (CTP)

CTP is a short peptide from the carboxyl terminus of the human chorionic gonadotropin (hCG) beta subunit. Four kinds of reproduction-related polypeptide hormones, follicle stimulating hormone (FSH), luteinizing hormone (LH), thyroid stimulating hormone (TSH), and human chorionic gonadotropin (hCG) contain the same alpha subunit and their respective specific beta subunits. Compared with the other three hormones, hCG has a significantly prolonged *in vivo* half-life, which is mainly due to the specific carboxyl terminal peptide (CTP) on the hCG β-subunit (Fares FA et al., Proc Natl Acad Sci USA, 1992, 89: 4304-4308). The native CTP contains 37 amino acid residues, including four O-glycosylation sites, and sialic acid residues at the terminus. The highly sialylated, negatively charged CTP can resist the clearance by the kidney, thereby prolonging the *in vivo* half-life of the protein. The present inventors creatively connect at least one CTP peptide with a flexible peptide linker having an appropriate length to form a new peptide linker, for linking hFGF21 to a fusion ligand *e.g.,* an immunoglobulin Fc fragment.

The present inventors have found that the N-terminal and C-terminal sequences of hFGF21 play critical roles in its function. The hFGF21 has a complex and fragile spatial conformation which makes itself poorly stable, easily degradable and easy to polymerize. The steric hindrance effect of the fusion ligand linked to the hFGF21 will interfere with the correct folding of hFGF21, resulting in a significant decrease or even loss of activity, or higher probability of producing a polymer. The addition of a CTP rigid unit between hFGF21 and an Fc variant is equivalent to the addition of a rigid peptide linker. On one hand, the addition of the CTP peptide ensures that the N-terminally fused hFGF21 does not affect the binding site in Fc variant for FcRn, thus having no effect on the half-life. In addition, the protein A binding site in Fc is important for purification steps. The addition of a CTP rigid unit ensures that the N-terminally fused hFGF21 will not "cover" its binding site for protein A. On the other hand, the addition of a CTP rigid unit prevents the Fc fragment having a size of about 25 kD from interfering with the correct folding of the N-terminally fused hFGF21, thus leading to no loss or decline of the biological activity/function of the hFGF21.

We have also demonstrated that simply prolonging the length of the flexible linker does not significantly relief the effect of the Fc fragment on hFGF21 activity. An over-length linker peptide can result in the generation of polymers and increased sensitivity to proteases, making hFGF21 susceptible to degradation. However, the addition of a CTP rigid unit makes the fusion protein more stable. This might because that the rigid CTP peptide containing multiple glycosyl side chains can form a stable steric conformation compared to the random coil of a flexible linker such as (GGGGS)n. This "block" effect causes the hFGF21 and Fc fragment to fold independently into correct three-dimensional conformations without affecting the biological activities of each other. Moreover, the protective effect of the glycosyl side chains of CTP reduces the sensitivity of the peptide linker to proteases, such that the fusion protein is less susceptible to degradation in the linking region. Furthermore, CTP is derived from native human hCG and is not immunogenic, and thus is more suitable for use as a linker peptide than a non-naturally encoded amino acid sequence.

### IgG Fc variants

### Non-lytic Fc variants

The Fc element is derived from the constant region (Fc fragment) of immunoglobulin IgG, and plays an important role in eradicating pathogens in immune defense. The Fc-mediated effector functions of IgG function through two mechanisms as follows: (1) After binding to Fc receptors (FcyRs) on the cell surface, pathogens are broken down by phagocytosis or lysis or by killer cells through the antibody-dependent cell-mediated cytotoxicity (ADCC) pathway. (2) Alternatively, after binding to C1q of the first complement component C1, the complement-dependent cytotoxicity (CDC) pathway is triggered and thus pathogens are lysed. Among the four subtypes of human IgG, IgG₁ and IgG₃ are able to bind to FcyRs effectively, and IgG₄ has lower binding affinity for FcyRs. The binding of IgG₂ to FcyRs is too low to be measured, so human IgG₂ has little ADCC effects. In addition, human IgG₁ and IgG₃ can also effectively bind to C1q to activate the complement cascade. Human IgG₂ binds weakly to C1q and IgG₄ does not bind to C1q (Jefferis R et al., Immunol Rev, 1998, 163: 59-76), so the CDC effect of human IgG₂ is also weak. Obviously, none of the native IgG subtypes is well suitable for constructing hFGF21/Fc fusion proteins. In order to obtain non-lytic Fc variants without effector functions, the most effective method is to mutate the complement- and receptor-binding regions of the Fc segment and adjust the binding affinity of Fc for related receptors to reduce or eliminate the ADCC and CDC effects and retain only the long *in vivo* half-life of Fc without the generation of cytotoxicity. More mutation sites contained in non-lytic Fc variants can be found in Shields RL et al., J Biol Chem, 2001,276(9):6591-604 or China Patent No. CN 201280031137.2.

### Fc variants with enhanced affinity to the neonatal receptor FcRn

The plasma half-life of IgG depends on its binding to FcRn. Typically, IgG binds to FcRn at pH 6.0 and dissociates from FcRn at pH 7.4 (plasma pH). Through the study on the binding sites of the two, the sites on IgG that bind to FcRn are modified to increase the binding affinity at pH 6.0. It has been proven that mutations of some residues in the human Fcγ domain, which are important for the binding to FcRn, can increase the serum half-life. Mutations in residues T250, M252, S254, T256, V308, E380, M428 and N434 have been reported to increase or decrease the FcRn-binding affinity (Roopenian et al., Nat.Review Immunology7:715-725,2007). Trastuzumab (Herceptin, Genentech) variants, disclosed in Korean Patent No. KR 10-1027427, show increased FcRn-binding affinity, and these variants contain one or more amino acid modifications selected from the group consisting of 257C, 257M, 257L, 257N, 257Y, 279Q, 279Y, 308F and 308Y. Bevacizumab (Avastin, Genentech) variants, provided in Korean Patent No. KR 2010-0099179, show prolonged *in vivo* half-life and these variants contain amino acid modifications N434S, M252Y/M428L, M252Y/N434S and M428L/N434S. In addition, Hinton et al. also found that two variants T250Q and M428L increased the binding affinity for FcRn by 3 and 7 times, respectively. When the two sites were mutated simultaneously, the binding affinity was increased by 28 times. In rhesus macaque, the M428L or T250Q/M428L variant shows a 2-fold increase in plasma half-life (Paul R. Hinton et al., J Immunol, 2006, 176:346-356). More mutation sites contained in Fc variants with increased binding affinity for FcRn can be found in China Patent No. CN201280066663.2. In addition, studies show that the T250Q/M428L mutations in the Fc regions of five humanized antibodies improve the interaction between the Fc domain and FcRn. Moreover, in subsequent *in vivo* pharmacokinetic tests, compared to wild-type antibodies, the Fc mutated antibodies show improved pharmacokinetic parameters, such as increased *in vivo* exposure, reduced clearance, and increased subcutaneous bioavailability, when administered via subcutaneous injection (Datta-Mannan A et al., MAbs. Taylor & Francis, 2012, 4(2) :267-273.).

The term "disorders associated with severe inactivating mutations in the insulin receptor" describes conditions in subjects with mutations in the insulin receptor (or possible proteins directly downstream from it) which cause severe insulin resistance but are often occurred without the obesity commonly found in type 2 diabetes. In many ways, subjects afflicted with these conditions manifest hybrid symptoms of type 1 diabetes and Type 2 diabetes. Subjects thereby afflicted fall into several categories of roughly increasing severity, including: Type A Insulin Resistance, Type C Insulin Resistance (AKA HAIR-AN Syndrome), Rabson-Mendenhall Syndrome and Donohue's Syndrome or Leprechaunism. These disorders are associated with very high endogenous insulin levels, resulting in hyperglycemia. Subjects thereby afflicted also present with various clinical features associated with "insulin toxicity," including hyperandrogenism, polycystic ovarian syndrome (PCOS), hirsuitism, and acanthosis nigricans (excessive growth and pigmentation in the folds of the skin).

"Diabetic complications" are dysfunctions in other parts of the body caused by chronic hyperglycemia such as diabetic nephropathy, diabetic neuropathy, diabetic feet (foot ulcers and poor circulation), and ocular lesions (retinopathies). Diabetes also increases the risk for heart disease and bone and joint disorders. Other long-term complications of diabetes include skin problems, digestive problems, sexual dysfunction and problems with teeth and gums.

"Metabolic syndrome (MS)" is a pathological condition in which a variety of metabolic components are abnormally aggregated, including: (1) abdominal obesity or overweight; (2) atherosclerosis and dyslipidemia, such as hypertriglyceridemia (TG) and low high-density lipoprotein cholesterol (HDL-C); (3) hypertension; (4) insulin resistance and/or abnormal glucose tolerance. Some criteria also include microalbuminuria, hyperuricemia, increased pro-inflammatory state (C-reactive protein, CRP), and increased pro-thrombotic state (increased fibrinogen and plasminogen inhibitor-1, PAI-1).

"Dyslipidemia" refers to a disorder of lipoprotein metabolism, including lipoprotein overproduction or deficiency. Dyslipidemias may be manifested by an increase in the total cholesterol, low-density lipoprotein (LDL) cholesterol and triglyceride concentrations, and a decrease in high-density lipoprotein (HDL) cholesterol concentration in the blood.

"Non-alcoholic fatty liver disease (NAFLD)" is a spcetrum of liver disease characterized by hepatic steatosis, in the absence of excessive alcohol consumption.

"Non-alcoholic steatohepatitis (NASH)" is a spectrum of liver disease that includes simple steatosis, lobular inflammation, progressing to fibrosis, in the absence of excessive alcohol consumption..

### BRIEF DESCRIPTION OF DRAWINGS

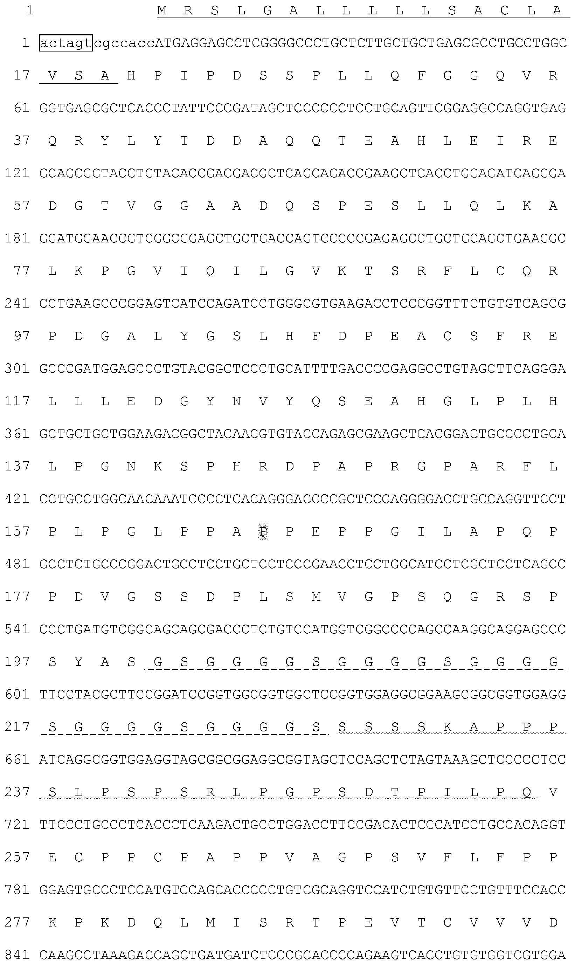

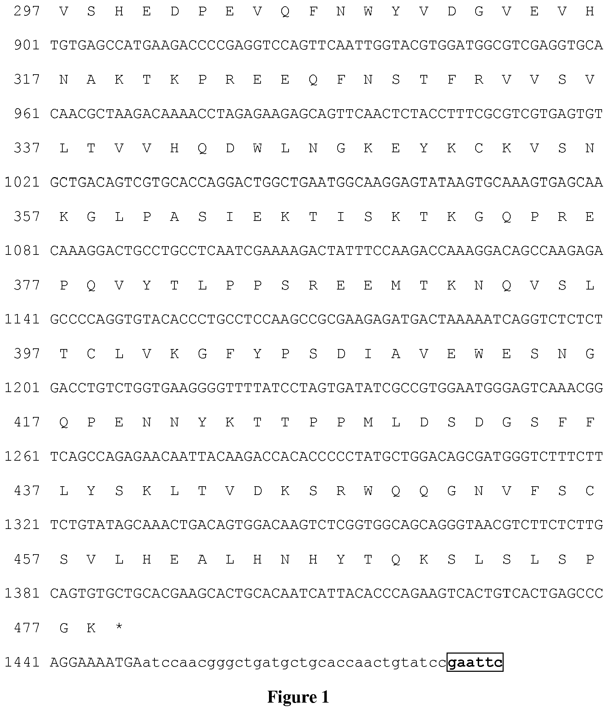

Figure 1 shows the nucleotide sequence and deduced amino acid sequence of the hFGF21 fusion protein inserted into SpeI/EcoRI (restriction sites are marked with 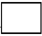 ) fragment in the PCDNA3.1 expression vector according to an embodiment of the present invention, consisting of a α1 microglobulin lead peptide (1-19, marked as 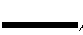 ), hFGF21 (20-200, having P instead of L), a flexible peptide linker (201-227, marked as 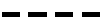 ), CTP¹ (228-255, marked as 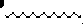 ), and vFc (256-478).

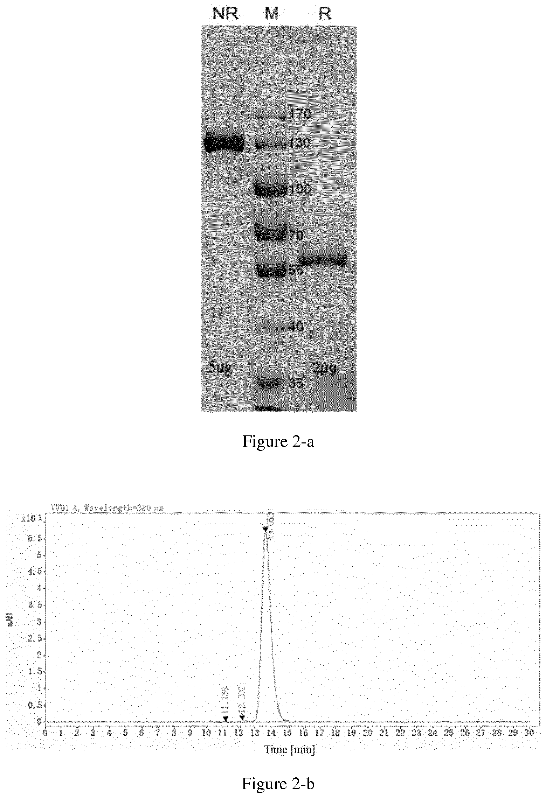

Figure 2-a. Results of reducing and non-reducing SDS-PAGE electrophoresis for hFGF21 fusion protein FP4I, from left to right bands: NR (non-reducing); M (protein Marker); and R (reducing).
Figure 2-b. SEC-HPLC results for hFGF21 fusion protein FP4I.
Figure 3-a. Curve of the glucose tolerance test at 16 hours after a single injection of hFGF21 fusion protein FP4I (means ± SEM, n=8).

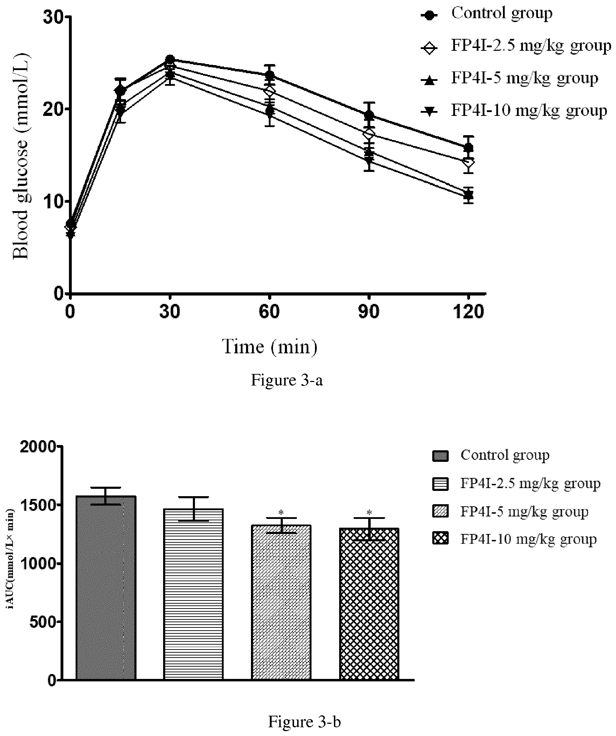

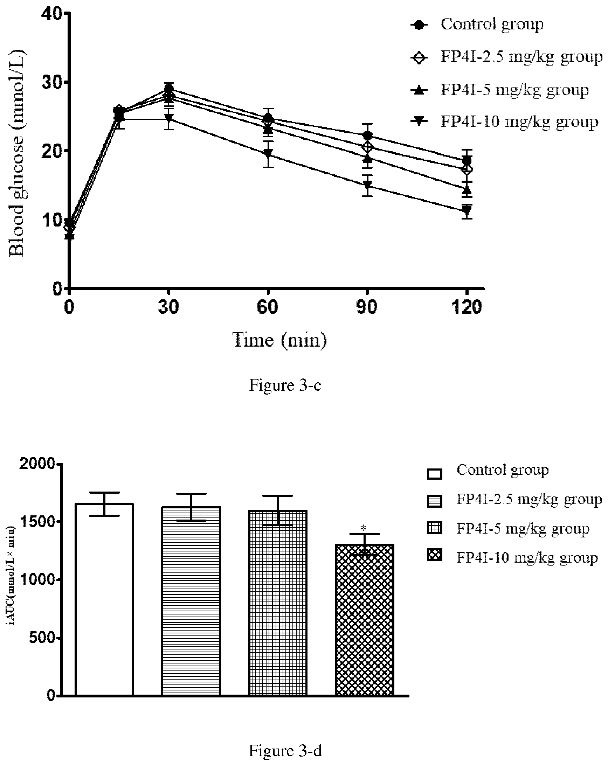

Figure 3-b. iAUC of the glucose tolerance test at 16 hours after a single injection of hFGF21 fusion protein FP4I (means ± SEM, n=8). Notes on statistical difference markers: **P*< 0.05 compared to control group.
Figure 3-c. Curve of the glucose tolerance test at 48 hours after a single injection of hFGF21 fusion protein FP4I (means ± SEM, n=8).
Figure 3-d. iAUC of the glucose tolerance test at 48 hours after a single injection of hFGF21 fusion protein FP4I (means ± SEM, n=8). Notes on statistical difference markers: **P*< 0.05 compared to control group.

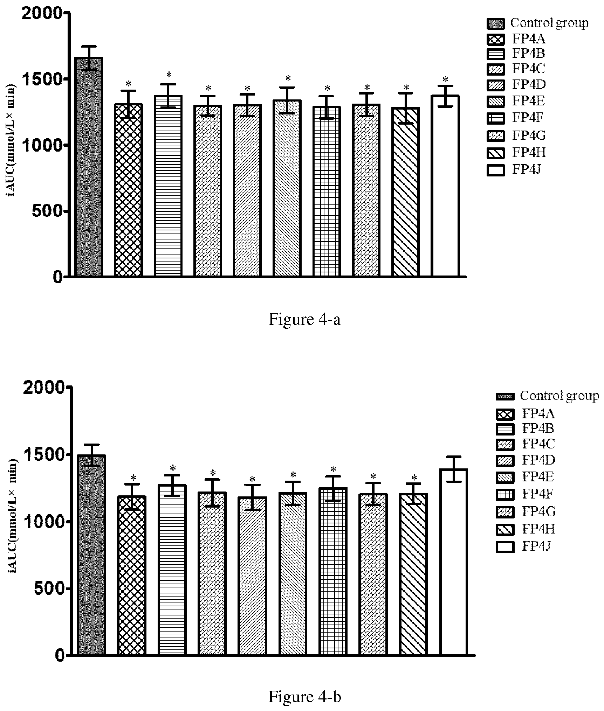

Figure 4-a. iAUC of the glucose tolerance test at 16 hours after a single injection of several hFGF21 fusion proteins (means ± SEM, n=8). Notes on statistical difference markers: **P*<0.05 compared to control group.
Figure 4-b. iAUC of the glucose tolerance test at 48 hours after a single injection of several hFGF21 fusion proteins (means ± SEM, n=8). Notes on statistical difference markers: **P*<0.05 compared to control group.

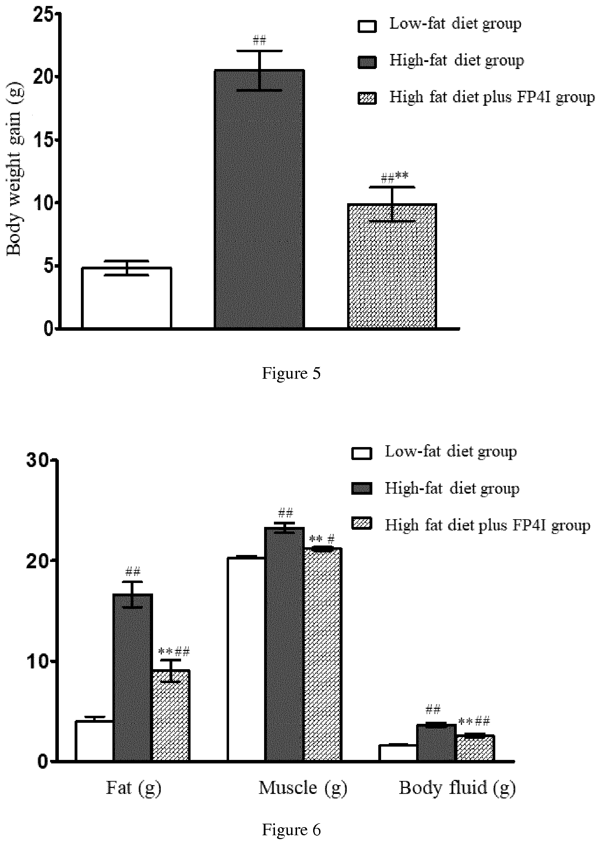

Figure 5. Effect of hFGF21 fusion protein FP4I on body weight gain in mice fed with high-fat diet (means ± SEM, n=8). Notes on statistical difference markers: ^{#}*P*<0.05, ^{##}*P*<0.01 compared to the low-fat diet group; **P*<0.05, ***P*<0.01 compared to the high-fat diet group.
Figure 6. Effect of hFGF21 fusion protein FP4I on body composition of mice fed with high-fat diet (means ± SEM, n=8). Notes on statistical difference markers: ^{#}*P*<0.05, ^{##}*P*<0.01 compared to the low-fat diet group; **P*<0.05, ***P*<0.01 compared to the high-fat diet group.

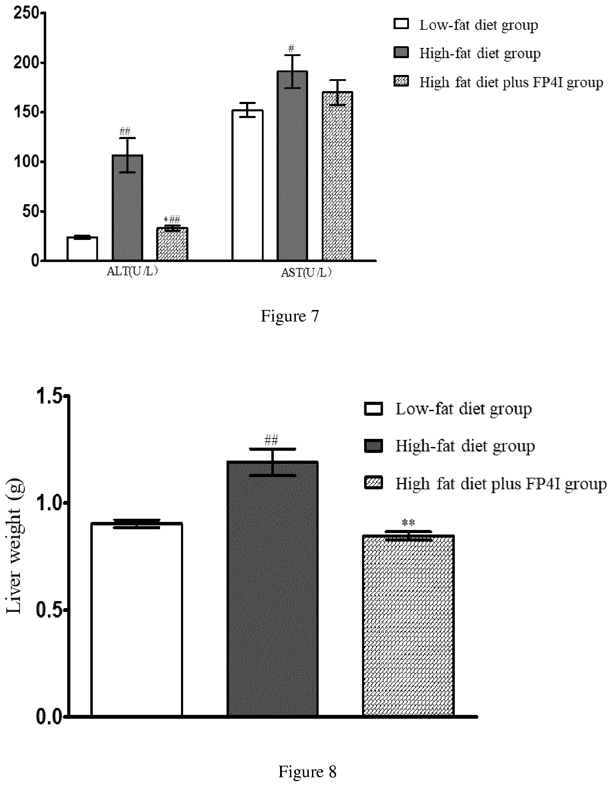

Figure 7. Effect of hFGF21 fusion protein FP4I on liver function in mice fed with high-fat diet (means ± SEM, n=8). Notes on statistical difference markers: ^{#}*P*<0.05, ^{##}*P*<0.01 compared to the low-fat diet group; **P*<0.05, ***P*<0.01 compared to the high-fat diet group.
Figure 8. Effect of hFGF21 fusion protein FP4I on liver weight of mice fed with high-fat diet (means ± SEM, n=8). Notes on statistical difference markers: ^{#}*P*<0.05, ^{##}*P*<0.01 compared to the low-fat diet group; **P*<0.05, ***P*<0.01 compared to the high-fat diet group.

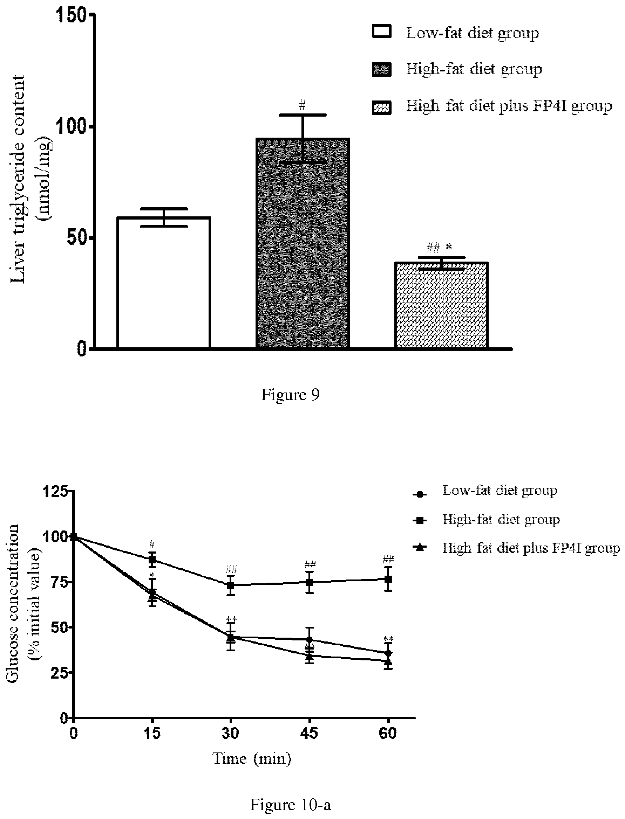

Figure 9. Effect of hFGF21 fusion protein FP4I on liver triglyceride content in mice fed with high-fat diet (means ± SEM, n=8). Notes on statistical difference markers: ^{#}*P*<0.05, *^{##}P*< 0.01 compared to the low-fat diet group; **P*<0.05, ***P*<0.01 compared to the high-fat diet group.

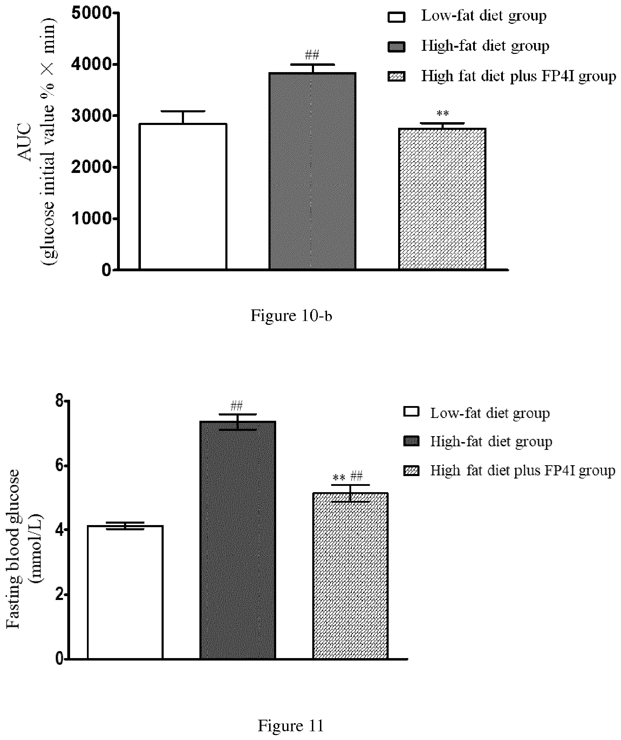

Figure 10-a. Blood glucose curve obtained from an experiment investigating the effect of hFGF21 fusion protein FP4I on insulin tolerance of mice fed with high-fat diet (means ± SEM, n=8). Notes on statistical difference markers: ^{#}*P*<0.05, *^{##}P*<0*.*01 compared to the low-fat diet group; **P*<0.05, ***P*<0.01 compared to the high-fat diet group.
Figure 10-b. AUC obtained from an experiment investigating the effect of hFGF21 fusion protein FP4I on insulin tolerance of mice fed with high-fat diet (means ± SEM, n=8). Notes on statistical difference markers: ^{#}*P*<0.05, ^{##}*P*<0.01 compared to the low-fat diet group; **P*<0.05, ***P*<0.01 compared to the high-fat diet group.
Figure 11. Effect of hFGF21 fusion protein FP4I on fasting blood glucose levels in mice fed with high-fat diet (means ± SEM, n=8). Notes on statistical difference markers: ^{#}*P*<0.05, *^{##}P*< 0.01 compared to the low-fat diet group; **P*<0.05, ***P*<0.01 compared to the high-fat diet group.

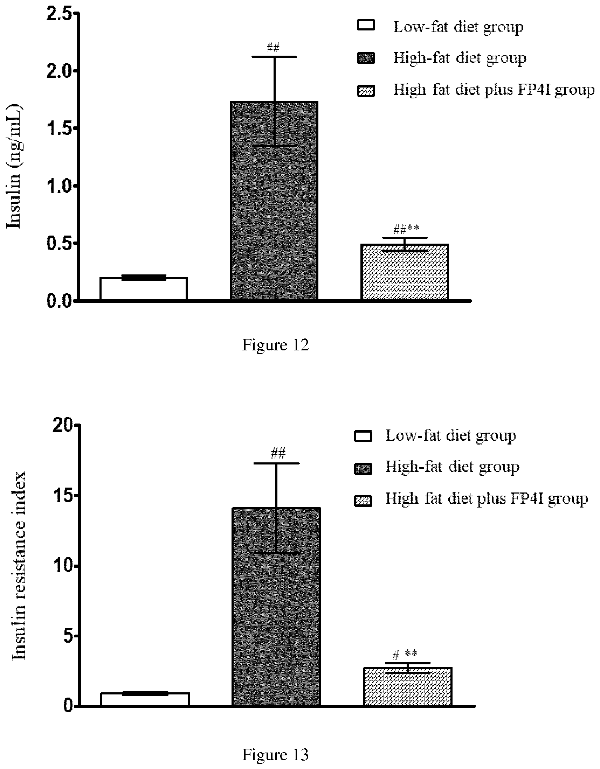

Figure 12. Effect of hFGF21 fusion protein FP4I on fasting insulin levels in mice fed with high-fat diet (means ± SEM, n=8). Notes on statistical difference markers: ^{#}*P*<0.05, ^{##}*P*<0.01 compared to the low-fat diet group; **P*<0.05, ***P*<0.01 compared to the high-fat diet group.
Figure 13. Effect of hFGF21 fusion protein FP4I on insulin resistance index in mice fed with high-fat diet (means ± SEM, n=8). Notes on statistical difference markers: ^{#}*P*<0.05, *^{##}P*< 0.01 compared to the low-fat diet group; **P*<0.05, ***P*<0.01 compared to the high-fat diet group.

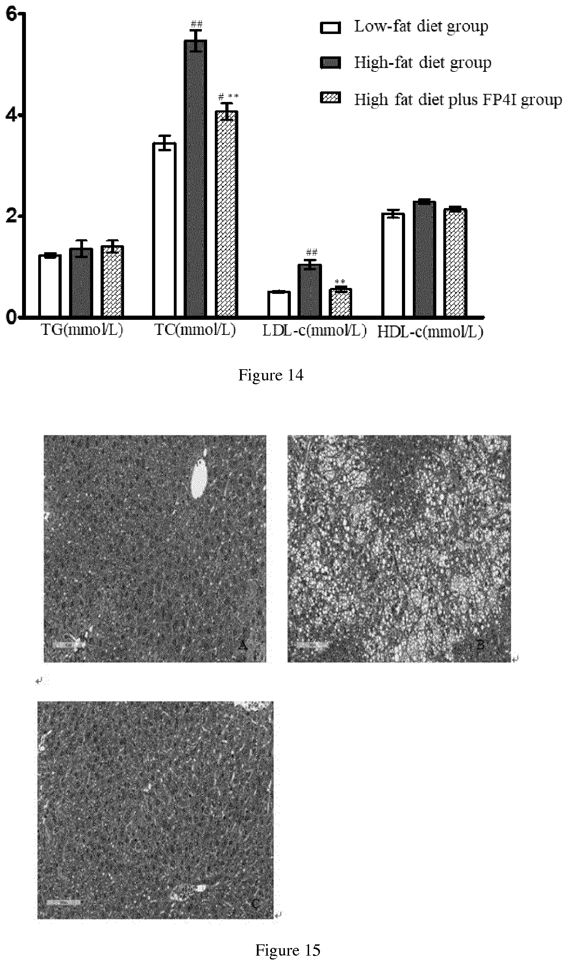

Figure 14. Effect of hFGF21 fusion protein FP4I on serum lipids in mice fed with high-fat diet (means ± SEM, n=8). Notes on statistical difference markers: ^{#}*P*<0.05, ^{##}*P*<0.01 compared to the low-fat diet group; **P*<0.05, ***P*<0.01 compared to the high-fat diet group.
Figure 15. Effect of hFGF21 fusion protein FP4I on the morphopathology of liver tissue in mice fed with high-fat diet. Notes: A: the low-fat diet group; B: the high-fat diet group; C: the group with high-fat diet combined with hFGF21 fusion protein.

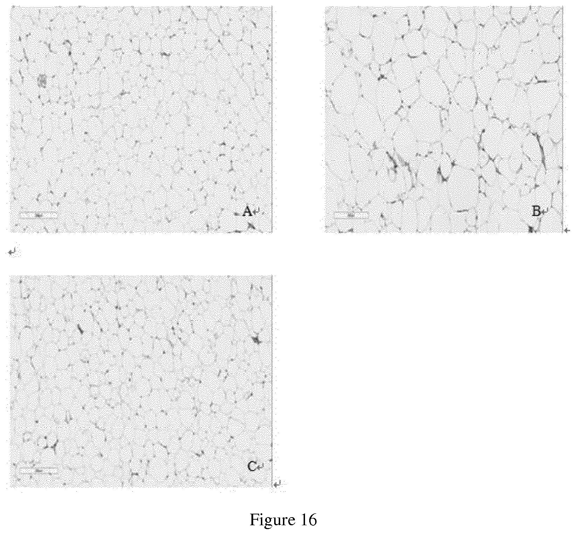

Figure 16. Effect of hFGF21 fusion protein FP4I on the morphopathology of adipose tissue in mice fed with high-fat diet. Notes: A: the low-fat diet group; B: the high-fat diet group; C: the group with high-fat diet combined with hFGF21 fusion protein.

### DETAILED DESCRIPTION

The present invention is further illustrated below in combination with specific embodiments. It should to be understood that the embodiments are only illustrative and are not intended to limit the scope of the present invention. In the following examples, experimental methods in which conditions are not specifically indicated are usually carried out according to conventional conditions, such as conditions described in Sambrook et al., Molecular Cloning: A Laboratory Manual (New York: Cold Spring Harbor Laboratory Press, 1989), or according to conditions suggested by the manufacturer.

The fusion proteins of the present invention are typically prepared by biosynthetic methods. Based on the nucleotide sequence of the present invention, one skilled in the art can conveniently prepare the nucleic acid of the present invention by various known methods, for example, but not limited to, PCR, DNA synthesis, etc. For specific methods, see J. Sambrook, Molecular Cloning: A Laboratory Manual. As an embodiment of the present invention, the nucleic acid sequence of the present invention can be constructed by segmentally synthesizing nucleotide sequences and then performing overlap extension PCR.

The present invention also provides an expression vector comprising a sequence encoding a fusion protein of the present invention and an expression regulatory sequence operably linked thereto. By "operably link" or "operably linked to" is meant a condition in which some portions of a linear DNA sequence are capable of regulating or controlling the activity of other portions of the same linear DNA sequence. For instance, a promoter is operably linked to a coding sequence if the promoter controls the transcription of the sequence.

The expression vector may be a commercially available vector such as, but not limited to, pcDNA3, pIRES, pDR, pUC18 or the like which can be used in a eukaryotic cell expression system. One skilled in the art can select a suitable expression vector based on the host cell.

The coding sequence of the fusion protein of the present invention may be introduced into suitable restriction sites by one skilled in the art by restriction enzyme cleavage and splicing according to a conventional method based on the restriction enzyme map of the known empty expression vector, to produce the recombinant expression vector of the present invention.

The invention also provides a host cell expressing a fusion protein of the invention comprising a coding sequence of a fusion protein of the present invention. The host cell is preferably a eukaryotic cell such as, but not limited to, CHO, COS cells, 293 cells, RSF cells and the like. In a preferred embodiment of the present invention, the cell is a CHO cell which can well express the fusion protein of the present invention to obtain a fusion protein having good binding activity and good stability.

The present invention also provides a method for producing a fusion protein of the present invention by using recombinant DNA, including the steps of:
1) providing a nucleic acid sequence encoding a fusion protein;
2) inserting the nucleic acid sequence of 1) into a suitable expression vector to obtain a recombinant expression vector;
3) introducing the recombinant expression vector of 2) into a suitable host cell;
4) growing the transformed host cell under conditions suitable for expression;
5) collecting the supernatant and purifying the fusion protein product.

The coding sequence can be introduced into a host cell by various techniques known in the art such as, but not limited to, calcium phosphate precipitation, protoplast fusion, lipofection, electroporation, microinjection, reverse transcription, phage transduction and method using alkali metal ions.

For the culture and expression of host cells, see Olander RM Dev Biol Stand, 1996, 86:338. The cells and debris in the suspension can be removed by centrifugation and the supernatant is collected. Identification can be performed by agarose gel electrophoresis.

The fusion protein obtained as described above can be purified to a substantially uniform nature, for example, showing a single band on SDS-PAGE electrophoresis. For example, when the recombinant protein is expressed by secretion, a commercially available ultrafiltration membrane such as products from Millipore, Pellicon, etc. can be used to separate the protein. The supernatant is firstly to be concentrated. The concentrated supernatant may be further purified by gel chromatography or by ion exchange chromatography, such as anion exchange chromatography (DEAE, etc.) or cation exchange chromatography. The gel matrix may be a matrix commonly used for protein purification such as agarose, dextran, polyamide, and the like. The Q- or SP- group is a preferred ion exchange group. Finally, the purified product may be further finely purified by methods such as hydroxyapatite adsorption chromatography, metal chelate chromatography, hydrophobic interaction chromatography and reversed-phase high performance liquid chromatography (RP-HPLC), and the like. All of the above purification steps can be used in different combinations to ultimately obtain proteins with a substantially uniform purity.

The expressed fusion protein can be purified by using an affinity chromatography column containing an antibody, receptor or ligand specific for the fusion protein. Depending on the nature of the affinity column used, the fusion polypeptide bound to the affinity column can be eluted by using conventional methods such as high salt buffer, pH change, and the like. Alternatively, the fusion protein may also contain one or more polypeptide fragments as a protein tag at the amino terminus or carboxyl terminus. Any suitable label can be used in the present invention. For example, the label may be FLAG, HA, HA1, c-Myc, 6-His or 8-His, and the like. These tags can be used to purify the fusion protein.

### Example 1. Construction of an expression plasmid encoding the hFGF21 Fc fusion protein

Gene sequences encoding the α1 microglobulin leader peptide and mature hFGF21 or its analog, flexible peptide linker, CTP rigid unit and human IgG Fc variant were artificially-optimized, CHO cell-biased codons. The full-length sequence was obtained by chemical synthesis. A restriction site, SpeI or EcoRI respectively, were present at each of the 5'-end and 3'-end of the synthesized fragment to facilitate insertion of the target fragment into a specific site of the expression vector. The verified gene of the fusion protein of hFGF21 or its analog was digested with SpeI and EcoRI, and then inserted into corresponding restriction sites in PCDNA3.1-modified plasmid PXY1A1 to obtain an expression plasmid of the fusion gene. This plasmid contains a cytomegalovirus early promoter which is an enhancer required for mammalian cells to express foreign genes at high levels. This plasmid also contains selectable markers which may confer kanamycin resistance to bacteria and G418 resistance to mammalian cells. In addition, the PXY1A1 expression vector contains the mouse dihydrofolate reductase (DHFR) gene, thus the fusion gene and DHFR gene may be co-amplified in the presence of methotrexate (MTX) in host cells deficient in expressing a DHFR gene (See U.S. Pat. No. 4,399,216).

As shown in Table 1, the present invention constructed a series of fusion proteins of hFGF21, which comprised wild-type hFGF21 and its analogs, flexible peptide linkers (Linkers) with different lengths and Fc variant (vFc) elements of different IgG subtypes. In addition, the position and length of the CTP rigid unit were also different. In order to verify that fusion proteins comprising at least one CTP rigid unit with different lengths have a high biological activity, we constructed fusion proteins FP4A, FP4B, FP4C, FP4D, FP4E, FP4F, FP4G, FP4H and FP4I; as well as FP4J comprising no CTP rigid unit. The nucleotide sequence and translated amino acid sequence of FP4I are shown in FIG. 1.

In the present example, the wild-type hFGF21 had the amino acid sequence as shown in SEQ ID NO: 1 (expressed as hFGF21), and it had an equivalent form, ^{L174P}hFGF21, wherein the Leu at position 174 of SEQ ID NO: 1 was replaced by Pro. The hFGF21 analog preferably comprises a truncated wild-type hFGF21 polypeptide, hFGF21(HPIP⁻), wherein the HPIP at the N-terminus of the wild-type hFGF21 mature protein has been deleted (having an amino acid sequence of amino acids 33-209 of SEQ ID NO: 1). The hFGF21 analog also preferably comprises variants in which an amino acid has been substituted/replaced, such as variant Q55C, which is obtained by replacing Gln at position 55 of SEQ ID NO: 1 with Cys, and several other variants including A109T, L126R K150R, P199G and G202A which are obtained by replacing the amino acid at the corresponding site of SEQ ID NO: 1.

**Table 1. Composition of the constructed hFGF21 fusion protein**

| Code | Elemental composition of a series of fusion proteins of hFGF21 and its analogs (from N-terminus to C-terminus) |
|---|---|
| FP4A | ^{P199G}hFGF21-L1-CTP¹-vFcγ₂₋₃ |
| FP4B | hFGF21(HPIP⁻)-L2-CTP¹-vFcγ₂₋₃ |
| FP4C | ^{K150R}hFGF21-L3-CTP⁵-vFcγ₁ |
| FP4D | ^{G202A}hFGF21-L4-CTP⁴-vFcγ₄ |
| FP4E | ^{A109T}hFGF21-L1-CTP³-CTP³-CTP³-vFcγ₂₋₂ |
| FP4F | hFGF21-L5-CTP⁵-CTP⁵-vFcγ₂₋₃ |
| FP4G | ^{Q55C}hFGF21-L4-CTP³-vFcγ₂₋₂ |
| FP4H | ^{L126R}hFGF21-L6-CTP²-vFcγ₄ |
| FP4I | ^{L174P}hFGF21-L3-CTP¹-vFcγ₂₋₃ |
| FP4J | ^{L174P}hFGF21-L3-vFcγ₂₋₃ |

### Example 2. Expression of fusion protein in transfected cell lines

The recombinant expression vector plasmid was transfected into a mammalian host cell line to express the hFGF21 fusion protein. DHFR enzyme-deficient CHO cells are preferred host cell line for stable expression at high levels (See U.S. Pat. No. 4,818,679). CHO-derived cell line DXB11 was used as a host cell in this example. A preferred method of transfection was electroporation, and other methods including calcium phosphate co-deposition and liposome transfection might also be used. In electroporation, Gene Pulser Electroporator (Bio-Rad Laboratories, Hercules, CA) was used at a voltage of 300 V and a capacitance of 1500 µFd, and 50 µg of high-purity expression plasmid was added to 5×10⁷ cells in the cuvette. Two days after the transfection, the medium was replaced with a growth medium containing 0.6 mg/mL of G418. Transfectants resistant to the selected drug were screened by an ELISA assay against human IgG Fc. Quantification of the expression of the fusion protein can also be performed by using an ELISA assay against hFGF21. The wells producing high levels of Fc fusion protein were subcloned by limiting dilutions on 96-well culture plates.

To achieve higher levels of fusion protein expression, co-amplification utilizing the DHFR gene that can be inhibited by an MTX drug is preferred. The transfected gene of the fusion protein was co-amplified with the DHFR gene in growth media containing increasing concentrations of MTX. Subclones with positive DHFR expression were subjected to limiting dilution, and transfectants capable of growing in media containing up to 6 µM of MTX were screened by increasing the selection pressure gradually. The transfectants were measured for secretion rates and cell lines yielding high levels of exogenous protein were screened. Cell lines with a secretion rate of more than about 30, preferably about 50 µg/10⁶ [*i.e.* million] cells/24h, were adapted to suspension culture by using serum-free growth media. Conditioned media was then used to purify the fusion protein.

### Example 3. Purification and characterization of the fusion protein

The conditioned media containing the fusion protein was titrated to pH 7∼8 with 1 N NaOH and then filtered through a 0.45 micron nitrocellulose filter. The filtrate was loaded onto a Protein A column that had been equilibrated with phosphate buffered saline (PBS). After the fusion protein was bound onto the Protein A column, the effluent fractions were discarded. The column was washed with PBS until the OD value at 280 nm was less than 0.01. The bound fusion protein was then eluted with 0.1 M citrate buffer (pH 3.75). The eluate was neutralized with 0.4 volume of 1 M K₂HPO₄, and fractions containing the purified protein were combined, dialyzed against PBS, and then filtered through a 0.22 micron nitrocellulose filter and stored at 4 °C. The protein product was identified and analyzed by SDS-PAGE under non-reducing and reducing conditions. The results of SDS-PAGE electrophoresis for purified FP4I protein were shown in Figure 2-a. Under non-reducing conditions, only one clear band (at approximately 130 KD) was present, without polymer. Under reducing conditions, only one band (at approximately 65 KD) was present, without degradation products. In addition, the purity of the fusion protein FP4I sample was examined by SEC-HPLC. As a result, as shown in Fig. 2-b, the percentage of the main peak area reached 98.87%, without polymer. The above results indicated that the fusion protein constructed by the present invention had a good stability and was not susceptible to degradation and aggregation.

### Example 4. Effect of a single injection of hFGF21 fusion protein on glucose utilization rate in obese mice induced by high-fat diet

32 male C57BL/6J mice aged 7 weeks (purchased from SLAC Laboratory Animal Co., Ltd, Shanghai, SPF-grade) were housed in a controlled environment: temperature of 22-25°C, relative humidity of 45-65%, and 12 h light/dark cycle. After a week of adaption, the mice were fed with high-fat diet (D12492 diet, a product from Research Diets, USA) for 12 weeks. The mice were divided into 4 groups according to the body weight: control group, FP4I-2.5 mg/kg (low-dose group), FP4I-5 mg/kg (middle-dose group) and FP4I-10 mg/kg (high-dose group). The mice in the drug-administration groups were subcutaneously injected with the corresponding FP4I solutions, and the mice in the control group were subcutaneously injected with PBS buffer. Thereafter, all the mice in each group were fasted for 16 hours followed by a glucose tolerance test. The fasting blood glucose level was measured. The mice were then intraperitoneally injected with 2 g/kg of glucose solution and the blood glucose levels at 15 min, 30 min, 60 min, 90 min and 120 min after injection were measured. The incremental area under the curve and above the baseline (iAUC) was calculated by trapezoidal method. 48 hours after drug administration, glucose tolerance test was carried out once more as described above. Data were expressed as mean ± standard error of mean (mean ± SEM) and analyzed using SPSS 18.0 statistical software. In normal distribution, differences between the means of multiple groups were analyzed by one-way ANOVA, followed by LSD test for homogeneous variances or Dunnett's T3 test for heterogeneous variances. Abnormal distribution was examined by non-parametric test. P values of less than 0.05 were considered statistically significant.

As show in Fig. 3a and Fig. 3b, middle- and high-dose of FP4I significantly improved the glucose utilization in obese mice at 16 h after administration when compared with the control group *(P<0.05).* As shown in Fig. 3c and Fig. 3d, the high dose of FP4I could significantly improve the glucose utilization level in obese mice at 48 h after administration as well *(P<0.05).* The data demonstrated that the hFGF21 fusion protein provided by the present invention had a long-lasting effect on improving glucose utilization.

The metabolic activities of other hFGF21 fusion proteins *in vivo* were evaluated by glucose tolerance test as described above. The obese mice induced by high-fat diet were randomly divided into 10 groups (n=6 each group): FP4A, FP4B, FP4C, FP4D, FP4E, FP4F, FP4G, FP4H, FP4J, and control group. Mice in drug-administration groups were subcutaneously injected with 10 mg/kg of corresponding protein solutions while the mice in the control group were subcutaneously injected with PBS buffer. Glucose tolerance tests were performed at 16 h and 48 h after injection, respectively. The results showed that FP4A, FP4B, FP4C, FP4D, FP4E, FP4F, FP4G, FP4H and FP4J significantly improved glucose utilization 16 h after drug-administration *(P<0.05)* (Figure 4a), and FP4A, FP4B, FP4C, FP4D, FP4E, FP4F, FP4G and FP4H significantly improved glucose utilization at 48 h after drug-administration *(P<0.05).* However, there was no significant difference in blood glucose level between the FP4J group and the control group (Fig. 4b). Additionally, the data indicated that the hFGF21 fusion protein provided by the present invention had a strong anti-proteolytic ability and was not easily degraded *in vivo,* thereby maintaining a long physiological activity. Furthermore, compared to the FP4J, a CTP-free fusion protein, the FP4I which contained a CTP structure showed a longer half-life in vivo, demonstrating that the presence of CTP can prolong the half-life *in vivo.*

### Example 5. Pharmacokinetic studies on a single dose of hFGF21 fusion protein in rats

Six male SD rats weighted 180 ± 10 g (purchased from SLAC Laboratory Animal Co., Ltd, Shanghai, SPF-grade) were housed in the following environment: temperature of 22-25°C, relative humidity of 45-65%, and 12 h light/dark cycle. After one week of adaption, 3 mg/kg of FP4I and FP4J were injected into the tail vein. 0.3 ml of blood was collected from the orbit respectively before administration, and at 1 h, 4 h, 7 h, 24 h, 48 h, 72 h, 96 h, 120 h, 149 h, 168 h, 192 h, 216 h and 240 h after administration. The whole blood was allowed to stand, and then centrifuged at 2000 × g for 15 minutes to obtain serum. The sandwich ELISA assay was performed to detect drug concentrations in serum. The mouse anti-hFGF21 antibody (R&D, Cat. No. MAB25373-100) was used as capturing antibody while HRP-labeled goat anti-human IgG-Fc mAb (Jackson, Inc., Cat. No. 109-035-098) was used as detecting antibody. The software PKSOLVER was applied to calculate the pharmacokinetic parameters: T_{1/2} and AUC₍₀₋ₜ₎.

The results showed that the circulating half-life T_{1/2} of FP4I at the dosage of 3mg/kg in SD rats was 29.81 ± 1.56 h, and the AUC₍₀₋ₜ₎ thereof was 1716711±201507 ng/ml·h, the circulating half-life T_{1/2} of FP4J at the dosage of 3mg/kg in SD rats was 22.43± 1.45 h, and the AUC₍₀₋ₜ₎ thereof was 1210604±191426 ng/ml·h, while the circulating half-life of native FGF-21 in rats was 1∼1.5 h. In summary, FP4I had a significantly increased half-life in vivo, and it had a longer circulating half-life in vivo and higher bioavailability than FP4J.

### Example 6. Preventive effect of hFGF21 fusion protein on obesity, nonalcoholic hepatic steatosis, insulin resisitance and hypercholesterolemia in obese mie induced by high-fat diet

### 1. Model establishment and drug administration

Twenty-four 7-week-old C57BL/6J male mice (purchased from Shanghai SLAC Laboratory Animal Co., Ltd) were housed in a controlled environment: temperature of 22-25°C, relative humidity of 45-65%, and 12 h light/dark cycle. After a week of adaption, the mice were divided into 3 groups according to their body weight: low-fat diet (LFD) group, high-fat diet (HFD) group, and high-fat diet plus 3.6 mg/kg hFGF21 fusion protein FP4I (HFD + FP4I 3.6 mg/kg) group. The mice in the LFD group and HFD groups were fed with D12450B diet and D12492 diet (Research diets, USA) respectively. Mice in the HFD + FP4I 3.6 mg/kg group were subcutaneously injected with 3.6 mg/kg of FP4I every four days, and mice in LFD and HFD groups were subcutaneously injected with PBS solution. The study was conducted for 116 days. At the end of the experiment, all the mice were fasted for 16 hours, and the fasting blood glucose levels and body weights were measured. Whole blood samples were collected by removing eyeballs, and then centrifuged at 2000×g for 15 min to separate and obtain the serum. The liver tissue was excised, washed with physiological saline, cleared the remaining fluid with filter paper and then weighed. Two parts of liver tissue were taken from the same site, wherein one was fixed in 10% formalin solution for HE staining, and the other was rapidly frozen in liquid nitrogen and stored at -80 °C for liver index analysis. The adipose tissue around the epididymis on one side was collected and fixed in a 10% formalin solution for HE staining.

### 2. Index detection

### 2.1. Body weight observation

The mice were weighed every 4 days, and muscle, fat and body fluid contents of the mice were analyzed by time-domain nuclear magnetic resonance on day 104 after the start of the experiment. Changes between body weight before and after the experiment were calculated according to the following formulae: body weight gain = the body weight of the mouse at the end of the experiment - the body weight of the mouse at the beginning of the experiment.

### 2.2. Serum biochemical analysis

ALT, AST, HDL-c, LDL-c, TG and TC concentrations in serum were measured with automatic biochemical analyzer (Erba XL-200). The specific procedure was performed according to the instrument manual.

### 2.3. Insulin tolerance test, fasting insulin level and insulin resistance index

At day 118, the mice in each group were fasted for 6 h (10:00 am - 4:00 pm) and then the basal blood glucose levels and body weight were measured. 0.75 IU/kg of insulin solution was injected intraperitoneally to the mice. The blood glucose levels were measured at 15, 30, 60, 90, 120 min after injection to draw an insulin tolerance curve. The AUC was calculated by trapezoidal method. The fasting serum insulin content in mice was measured by ELISA assay, and the insulin resistance index was calculated according to the following formula: insulin resistance index = fasting blood glucose level (mmol/L) × fasting insulin content (mIU/L)/22.5.

### 2.4. Liver triglyceride content measurement

50 mg of liver tissue was accurately excised for each mouse .The liver TG contents were measured following the method described by Floch and the results were expressed as the TG content per mg liver tissue.

### 2.5. Histopathology examination

Mouse liver and adipose tissues which were collected from the same site and preserved in 10% formalin solution were stained with HE for morphopathological observation.

### 3. Statistics and Analysis

Data were expressed as mean ± standard error of mean (mean ± SEM) and analyzed by using SPSS 18.0 statistical software. In normal distribution, differences between the means of multiple groups were analyzed by one-way ANOVA, followed by LSD test for homogeneous variances or Dunnett's T3 test for heterogeneous variances. Abnormal distribution was examined by non-parametric test. *P<0.05* indicated significant statistical difference.

### 4. Results

### 4.1. Effect of the hFGF21 fusion protein on body weight and fat content in mice fed with high-fat diet

Compared with the low-fat diet group, mice in the high-fat diet group had significantly increased body weight, body weight gain and body fat content, showing obvious obesity symptoms. The hFGF21 fusion protein significantly reduced the body weight gain and body fat content induced under high-fat diet feeding conditions. The results are shown in Figures 5-6.

### 4.2. Effect of the hFGF21 fusion protein on liver function and hepatic steatosis in mice fed with high-fat diet

As shown in Figure 7, compared with the low-fat diet group, serum ALT levels in mice of the high-fat diet group significantly increased, showing significant liver damage and lipid metabolism disorder. Compared to the high-fat diet group, the hFGF21 fusion protein significantly reduced serum ALT levels in mice. As shown in Fig. 8 and Fig. 9, the liver weight and liver triglyceride content in mice fed with high-fat diet were significantly increased, while the hFGF21 fusion protein significantly reduced the liver weight and liver triglyceride content in mice fed with high-fat diet.

### 4.3. Effect of hFGF21 fusion protein on insulin resistance in mice fed with high-fat diet.

The insulin tolerance test showed that mice fed high-fat diet showed significant symptoms of insulin resistance. Results of the serum insulin test further indicated that mice developed hyperinsulinemia and insulin resistance. The hFGF21 fusion protein significantly alleviated the insulin resistance symptoms and the increase in fasting blood glucose in mice fed with high-fat diet. The results are shown in Figures 10-13.

### 4.4 Effect of hFGF21 fusion protein on hypercholesterolemia in mice fed with high-fat diet

As shown in Figure 14, compared with the low-fat diet group, serum TC and LDL-c levels in mice of the high-fat diet group significantly increased, showing lipid metabolism disorder. Compared to the high-fat diet group, the hFGF21 fusion protein significantly reduced serum TC and LDL-c levels in mice.

### 4.5. Pathological morphology test

As shown in Figures 15 and 16, compared with the low-fat diet group, significant steatosis occurred in the liver tissue, the lipid droplet vacuoles were clearly visible and fused into a sheet-like structure, the morphology of liver cells was severely damaged, and the cross-sectional area of fat cells was significantly increased in mice of the high-fat diet group. Compared with the high-fat diet group, the hFGF21 fusion protein significantly attenuated the steatosis of liver tissue and reduced the cross-sectional area of fat cells.

Based on the above results, it was confirmed that hFGF21 fusion protein can effectively control the body weight, improve insulin resistance, reverse liver steatosis and hypercholesterolemia in obese mice induced by high-fat diet.

All documents mentioned in the present invention are hereby incorporated by reference to the same extent as if each of the documents is individually recited for reference. It is to be understood that various changes and modifications may be made by those skilled in the art upon reading the above teachings of the present invention, which also fall within the scope of the claims appended hereto.

## Claims

1. A human fibroblast growth factor 21 fusion protein comprising, in order from N-terminus to C-terminus, wild-type hFGF21 or an analog thereof, a flexible peptide linker, at least one rigid unit comprising the carboxyl terminal peptide of human chorionic gonadotropin β subunit, and a fusion ligand; wherein the fusion ligand is selected from the group consisting of an immunoglobulin and an Fc fragment thereof, human serum albumin and transferrin, preferably, the fusion ligand is an immunoglobulin Fc fragment.

2. The fusion protein of claim 1, wherein the fusion protein is glycosylated; preferably, the fusion protein is glycosylated by expressing the same in mammalian cells; more preferably, the fusion protein is glycosylated by expressing the same in Chinese hamster ovary cells.

3. The fusion protein of claim 1, wherein the wild-type hFGF21 comprises a sequence as shown in SEQ ID NO: 1 from which amino acids 1-28 (a leader peptide) are removed; or an isoform of the sequence as shown in SEQ ID NO: 1 from which amino acids 1-28 (a leader peptide) are removed and having G141S or L174P substitution.

4. The fusion protein of claim 1, wherein the hFGF21 analog has one or more amino acid deletions, insertions, additions or substitutions, and one or more amino acid deletions at the N-terminus or C-terminus relative to the amino acid sequence of wild-type hFGF21; preferably, the amino acid sequence of the hFGF21 analog is at least 70% homologous to that of wild-type hFGF21; more preferably, the amino acid sequence of the hFGF21 analog is at least 80% homologous to that of wild-type hFGF21; more preferably, the amino acid sequence of the hFGF21 analog is at least 90% homologous to that of wild-type hFGF21; and most preferably, the amino acid sequence of the hFGF21 analog is at least 95% homologous to that of wild-type hFGF21.

5. The fusion protein of claim 4, wherein the hFGF21 analog has 1, 2, 3, 4, 5, 6, 7, or 8 amino acid residues deleted at the N-terminus.

6. The fusion protein of claim 5, wherein the hFGF21 analog has 4 amino acid residues, HPIP, deleted at the N-terminus.

7. The fusion protein of claim 4, wherein the hFGF21 analog has 1, 2, 3, 4, 5, 6, 7, 8, 9, 10, 11 or 12 amino acid residues deleted at the C-terminus.

8. The fusion protein of claim 4, wherein the hFGF21 analog comprises one or more amino acid substitutions selected from the group consisting of Q55C, A109T, L126R, G148C, K150R, P158S, S195A, P199G and G202A.

9. The fusion protein of claim 1, wherein the flexible peptide linker contains two or more amino acids selected from the group consisting of G, S, A and T.

10. The fusion protein of claim 9, wherein the flexible peptide linker has the structural formula of (GS)ₐ(GGS)_{b}(GGGS)_{c}(GGGGS)_{d}, wherein each a, b, c, and d is an integer equal to or greater than 0, and a+b+c+d≥1.

11. The fusion protein of claim 10, wherein the flexible peptide linker has an amino acid sequence selected from the group consisting of:
(i) GGGGS;
(ii) GSGGGSGGGGSGGGGS;
(iii) GSGGGGSGGGGSGGGGSGGGGSGGGGS;
(iv) GGGGSGGGGSGGGGSGGGGSGGGGSGGGGSGGGGS;
(v) GGGSGGGSGGGSGGGSGGGS;
(vi) GGSGGSGGSGGS.

12. The fusion protein of claim 1, wherein the rigid unit comprising the carboxyl terminal peptide of human chorionic gonadotropin β subunit comprises SEQ ID NO: 2 or a truncated sequence thereof; wherein the truncated sequence comprises at least 2 glycosylation sites.

13. The fusion protein of claim 12, wherein the rigid unit comprising the carboxyl terminal peptide of human chorionic gonadotropin β subunit comprises the following amino acid sequences:
(i) SSSSKAPPPSLPSPSRLPGPSDTPILPQ;
(ii) PRFQDSSSSKAPPPSLPSPSRLPGPSDTPILPQ;
(iii) SSSSKAPPPS;
(iv) SRLPGPSDTPILPQ;
(v) SSSSKAPPPSLPSPSR.

14. The fusion protein of claim 1, wherein the amino acid sequence of the rigid unit comprising the carboxyl terminal peptide of human chorionic gonadotropin β subunit shares at least 70%, 80%, 90% or 95% identity to that of the CTP in the fusion protein of claim 13.

15. The fusion protein of claim 1, wherein the fusion protein comprises 1, 2, 3, 4 or 5 rigid units comprising the carboxyl terminal peptide of human chorionic gonadotropin β subunit.

16. The fusion protein of claim 1, wherein the human immunoglobulin Fc fragment is a variant having a reduced ADCC effect and/or CDC effect and/or an enhanced binding affinity to an FcRn receptor.

17. The fusion protein of claim 16, wherein the Fc fragment is selected from a human IgG Fc variant; more preferably, the human IgG Fc variant is selected from the group consisting of:
(i) hinge, CH2 and CH3 regions of human IgG1 containing mutations Leu234Val, Leu235Ala and Pro331Ser;
(ii) hinge, CH2 and CH3 regions of human IgG2 containing mutation Pro331Ser;
(iii) hinge, CH2 and CH3 regions of human IgG2 containing mutations Thr250Gln and Met428Leu;
(iv) hinge, CH2 and CH3 regions of human IgG2 containing mutations Pro331Ser, Thr250Gln and Met428Leu;
(v) hinge, CH2 and CH3 of human IgG4 regions containing mutations Ser228Pro and Leu235Ala.

18. The fusion protein of claim 1, wherein the fusion protein has the amino acid sequence as shown in SEQ ID NO: 8.

19. A DNA molecule encoding the fusion protein of any one of claims 1-18.

20. The DNA molecule of claim 19, wherein the DNA molecule comprises the sequence as shown in SEQ ID NO:9.

21. A vector comprising the DNA molecule of claim 19 or 20.

22. A host cell comprising the vector of claim 21, or transfected with the vector comprising the DNA molecule of claim 19 or 20.

23. A pharmaceutical composition comprising a pharmaceutically acceptable carrier, excipient or diluent, and an effective amount of the fusion protein of any one of claims 1-18.

24. A method for preparing the fusion protein of any one of claims 1-18, comprising:
(a) introducing the DNA sequence encoding the fusion protein of claim 19 or 20 into a mammalian cell;
(b) screening the high-yielding cell line in step (a) which expresses more than 50 µg/10⁶ (million) cells per 24 hours in its growth medium;
(c) growing the cell line obtained in step (b) to express the fusion protein;
(d) harvesting the fermentation broth obtained in step (c) and purifying the fusion protein;
preferably, the mammalian cell used in step (a) is a CHO cell; more preferably, the mammalian cell is CHO-derived cell line DXB-11.

25. Use of the fusion protein of any one of claims 1-18 for the manufacture of a medicament for the treatment of obesity, type 1 or type 2 diabetes, pancreatitis, dyslipidemia, non-alcoholic fatty liver disease (NAFLD), non-alcoholic steatohepatitis (NASH), insulin resistance, hyperinsulinemia, glucose intolerance, hyperglycemia, metabolic syndrome, acute myocardial infarction, hypertension, cardiovascular disease, atherosclerosis, peripheral arterial disease, stroke, heart failure, coronary heart disease, kidney disease, diabetic complications, neuropathy, and disorders associated with severe inactivation or mutation in the insulin receptor.
